# EUROPEAN PATENT APPLICATION

(11) **EP 3 505 162 A1**
(43) Date of publication of application: **03.07.2019**
(21) Application number: 18197869.3
(22) Date of filing: 11.08.2011
(51) Int. Cl.: A61K 9/20, A61K 31/485

(54) **USE OF BINDERS FOR MANUFACTURING STORAGE STABLE FORMULATIONS**

(30) Priority: 13.08.2010 US 37334410 P; 13.08.2010 EP 10172759
(62) Divisional of application: 11743537.0
(71) Applicant: Euro-Celtique S.A., 2350 Luxembourg (LU)
(72) Inventor: PRATER, Derek Allan, Milton Cambridge, Cambridgeshire CB24 6YS (GB); SPITZLEY, Christof, 65627 Elbtal (DE); HEUN, Gerhard Josef, 65611 Brechen (DE); SCHÜTZ, Alexander, 65597 Hünfelden Nauheim (DE); HAHN, Udo, 56412 Nentershausen (DE); LEUNER, Christian, 60439 Frankfurt (DE)
(74) Representative: Ledl, Andreas

(57) **Abstract**

The present invention relates to storage stable prolonged release pharmaceutical dosage forms comprising oxycodone or a pharmaceutically acceptable salt thereof and naloxone or a pharmaceutically acceptable salt thereof, the manufacture thereof as well as their use for administration to human beings.

## Description

### FIELD OF THE INVENTION

The present invention relates to the use of binders for manufacturing pharmaceutical dosage forms comprising oxycodone or a pharmaceutically acceptable salt thereof and naloxone or a pharmaceutically acceptable salt which are storage stable over prolonged periods of time.

### BACKGROUND OF THE INVENTION

In the manufacture of pharmaceutical compositions attention has to be paid to various aspects.

Prolonged release pharmaceutical compositions should release the pharmaceutically active ingredient(s) in a reproducible manner. At the same time, the release profile should be maintainable over prolonged periods of time to ensure that a patient will enjoy the therapeutic effect of pharmaceutical compositions over the course of the shelf life of such compositions.

There is a constant need for understanding how stability of pharmaceutical dosage forms is influenced and how it can be beneficially affected.

### SUMMARY OF THE INVENTION

It is an objective of the present invention to provide pharmaceutical compositions comprising the pharmaceutically active ingredients oxycodone or a pharmaceutically acceptable salt thereof and naloxone or a pharmaceutically acceptable salt which are storage stable over prolonged periods of time.

These and other objectives as they will become apparent from the ensuing description are attained by the subject matter of the independent claims. Some of the preferred embodiments are referred to by the dependent claims.

The present invention is concerned with an oral prolonged release pharmaceutical composition comprising at least:
a) at least one prolonged release material;
b) at least oxycodone or a pharmaceutically acceptable salt or derivative thereof and naloxone or a pharmaceutically acceptable salt or derivative thereof; and wherein the
c) pharmaceutical composition after storage under stressed conditions has less than 4 % of total substances related to oxycodone or a pharmaceutically acceptable salt or derivative thereof and/or related to naloxone or a pharmaceutically acceptable salt or derivative thereof.

In some embodiments the composition has equal to or more than 0.05% and preferably equal to or more than 0.1% of total substances related to oxycodone or a pharmaceutically acceptable salt or derivative thereof and/or related to naloxone or a pharmaceutically acceptable salt or derivative thereof.

The present invention is also concerned with an oral prolonged release pharmaceutical composition comprising at least:
a) at least one prolonged release material;
b) at least oxycodone or a pharmaceutically acceptable salt or derivative thereof and naloxone or a pharmaceutically acceptable salt or derivative thereof; and wherein the
c) pharmaceutical composition after storage under stressed conditions has less than 0.5 % of known substances related to oxycodone or a pharmaceutically acceptable salt or derivative thereof and/or related to naloxone or a pharmaceutically acceptable salt or derivative thereof.

In some embodiments the composition has equal to or more than 0.01% and preferably equal to or more than 0.05% of known substances related to oxycodone or a pharmaceutically acceptable salt or derivative thereof and/or related to naloxone or a pharmaceutically acceptable salt or derivative thereof

The present invention is also concerned with an oral prolonged release pharmaceutical composition comprising at least:
a) at least one prolonged release material;
b) at least oxycodone or a pharmaceutically acceptable salt or derivative thereof and naloxone or a pharmaceutically acceptable salt or derivative thereof; and wherein the
c) pharmaceutical composition after storage under stressed conditions has less than 0.5 % of a known substance related to oxycodone or a pharmaceutically acceptable salt or derivative thereof and/or related to naloxone or a pharmaceutically acceptable salt or derivative thereof.

In some embodiments the composition has equal to or more than 0.01% and preferably equal to or more than 0.05% of a known substance related to oxycodone or a pharmaceutically acceptable salt or derivative thereof and/or related to naloxone or a pharmaceutically acceptable salt or derivative thereof. These values preferably relate to the known related substance noroxymorphone.

The present invention is also concerned with an oral prolonged release pharmaceutical composition comprising at least:
a) at least one prolonged release material;
b) at least oxycodone or a pharmaceutically acceptable salt or derivative thereof and naloxone or a pharmaceutically acceptable salt or derivative thereof; and wherein the
c) pharmaceutical composition after storage under stressed conditions releases the pharmaceutically active agents with substantially the same release rate as before subjecting the pharmaceutical composition to stressed conditions.

The present invention is concerned with an oral prolonged release pharmaceutical composition comprising at least:
a) at least one prolonged release material;
b) at least oxycodone or a pharmaceutically acceptable salt or derivative thereof and naloxone or a pharmaceutically acceptable salt or derivative thereof; and
c) at least one binder, which preferably is HPC.

Prolonged release pharmaceutical compositions may comprise oxycodone or a pharmaceutically acceptable salt thereof in an amount equivalent to 1 to 15 mg, preferably 1 to 9 mg of oxycodone HCl and naloxone or a pharmaceutically acceptable salt thereof in an amount equivalent to 0.5 to 7.5 mg, preferably 0.5 to 4.5 mg of naloxone HCl.

These compositions may preferably comprise the active ingredients in 2:1 ratio by weight.

The active ingredients may preferably be oxycodone HCl and naloxone HCl.

The pharmaceutical compositions may comprise these active ingredients in prolonged release matrix. In one embodiment, the pharmaceutical composition uses hydroxypropyl cellulose as a binder. In another embodiment, the pharmaceutical composition does not comprise hydroxypropyl cellulose and/or povidone as a binder. Alternatively, prolonged release pharmaceutical compositions may comprise oxycodone or a pharmaceutically acceptable salt thereof in an amount equivalent to 10 to 160 mg, preferably 16 to 160 mg of oxycodone HCl and naloxone or a pharmaceutically acceptable salt thereof in an amount equivalent to 5 to 80 mg, preferably 9 to 80 mg of naloxone HCl.

These compositions may preferably comprise the active ingredients in 2:1 ratio by weight.

The active ingredients may preferably be oxycodone HCl and naloxone HCl or the solvates thereof.

The pharmaceutical compositions may comprise these active ingredients in prolonged release matrix. In one embodiment, the pharmaceutical composition uses povidone as a binder. In another embodiment, the pharmaceutical composition does not comprise povidone as a binder.

In another embodiment the present invention relates to the use of a binder for manufacturing a prolonged release pharmaceutical composition comprising oxycodone or a pharmaceutically acceptable salt thereof and naloxone or a pharmaceutically acceptable salt thereof, wherein the pharmaceutical composition after storage under stressed conditions has less than 4 % of total substances related to oxycodone or a pharmaceutically acceptable salt or derivative thereof and/or related to naloxone or a pharmaceutically acceptable salt or derivative thereof. In some embodiments the composition has equal to or more than 0.05% and preferably equal to or more than 0.1% of total substances related to oxycodone or a pharmaceutically acceptable salt or derivative thereof and/or related to naloxone or a pharmaceutically acceptable salt or derivative thereof.

In another embodiment the present invention relates to the use of a binder for manufacturing a prolonged release pharmaceutical composition comprising oxycodone or a pharmaceutically acceptable salt thereof and naloxone or a pharmaceutically acceptable salt thereof, wherein the pharmaceutical composition after storage under stressed conditions has less than 0.5 % of known substances or a known substance related to oxycodone or a pharmaceutically acceptable salt or derivative thereof and/or related to naloxone or a pharmaceutically acceptable salt or derivative thereof. In some embodiments the composition has equal to or more than 0.01% and preferably equal to or more than 0.07% of known substances or a known substance related to oxycodone or a pharmaceutically acceptable salt or derivative thereof and/or related to naloxone or a pharmaceutically acceptable salt or derivative thereof. These values preferably relate to the known related substance noroxymorphone.

In yet another embodiment the present invention relates to the use of a binder for manufacturing a prolonged release pharmaceutical composition comprising oxycodone or a pharmaceutically acceptable salt thereof and naloxone or a pharmaceutically acceptable salt thereof, wherein the pharmaceutical composition after storage under stressed conditions releases the pharmaceutically active agents with substantially the same release rate as before subjecting the pharmaceutical composition to stressed conditions.

In yet another embodiment the present invention relates to the use of a binder in manufacturing a prolonged release pharmaceutical composition comprising oxycodone or a pharmaceutically acceptable salt thereof and naloxone or a pharmaceutically acceptable salt thereof. The binder preferably may be HPC.

In a further embodiment the present invention relates to the use of a binder, preferably HPC for stabilizing a prolonged release pharmaceutical composition comprising oxycodone or a pharmaceutically acceptable salt thereof and naloxone or a pharmaceutically acceptable salt thereof.

In a further embodiment the present invention relates to the use of HPC for stabilizing a prolonged release pharmaceutical composition comprising oxycodone or a pharmaceutically acceptable salt thereof and naloxone or a pharmaceutically acceptable salt thereof.

In these embodiments the pharmaceutical compositions may comprise oxycodone or a pharmaceutically acceptable salt thereof in an amount equivalent to 1 to 15, preferably 1 to 9 mg of oxycodone HCl and naloxone or a pharmaceutically acceptable salt thereof in an amount equivalent to 0.5 to 7.5, preferably 0.5 to 4.5 mg of naloxone HCl.

These compositions may preferably comprise the active ingredients in 2:1 ratio by weight.

The active ingredients may preferably be oxycodone HCl and naloxone HCl.

The pharmaceutical compositions may comprise these active ingredients in prolonged release matrix. In some embodiments the matrix may not comprise ethyl cellulose and/or stearyl alcohol as prolonged release matrix materials. In one embodiment, the pharmaceutical composition uses hydroxypropyl cellulose as a binder. In another embodiment, the pharmaceutical composition does not comprise hydroxypropyl cellulose and/or povidone as a binder.

Alternatively, in these embodiments the pharmaceutical compositions may comprise oxycodone or a pharmaceutically acceptable salt thereof in an amount equivalent to 10 to 160 mg, preferably 16 to 160 mg of oxycodone HCl and naloxone or a pharmaceutically acceptable salt thereof in an amount equivalent to 5 to 80 mg, preferably 8 to 80 mg of naloxone HCl.

These compositions may preferably comprise the active ingredients in 2:1 ratio by weight.

The active ingredients may preferably be oxycodone HCl and naloxone HCl.

The pharmaceutical compositions may comprise these active ingredients in prolonged release matrix. In some embodiments the matrix may not comprise ethyl cellulose and/or stearyl alcohol as prolonged release matrix materials. In one embodiment, the pharmaceutical composition uses povidone as a binder. In another embodiment, the pharmaceutical composition does not comprise povidone as a binder.

In a further embodiment the present invention relates to the use of hydroxypropyl cellulose for manufacturing a prolonged release pharmaceutical composition comprising oxycodone or a pharmaceutically acceptable salt thereof and naloxone or a pharmaceutically acceptable salt thereof, wherein the pharmaceutical composition after storage under stressed conditions has less than 4 % of total substances related to oxycodone or a pharmaceutically acceptable salt or derivative thereof and/or related to naloxone or a pharmaceutically acceptable salt or derivative thereof.

In yet another embodiment the present invention relates to the use of hydroxypropyl cellulose as a binder for manufacturing a prolonged release pharmaceutical composition comprising oxycodone or a pharmaceutically acceptable salt thereof and naloxone or a pharmaceutically acceptable salt thereof, wherein the pharmaceutical composition after storage under stressed conditions releases the pharmaceutically active agents with substantially the same release rate as before subjecting the pharmaceutical composition to stressed conditions.

In yet another embodiment the present invention relates to the use of hydroxypropyl cellulose as a binder in manufacturing a prolonged release pharmaceutical composition comprising oxycodone or a pharmaceutically acceptable salt thereof and naloxone or a pharmaceutically acceptable salt thereof.

In these embodiments the pharmaceutical compositions may comprise oxycodone or a pharmaceutically acceptable salt thereof in an amount equivalent to 1 to 15 mg, preferably 1 to 9 mg of oxycodone HCl and naloxone or a pharmaceutically acceptable salt thereof in an amount equivalent to 0.5 to 7.5mg, preferably 0.5 to 4.5 mg of naloxone HCl.

These compositions may preferably comprise the active ingredients in 2:1 ratio by weight.

The active ingredients may preferably be oxycodone HCl and naloxone HCl.

The pharmaceutical compositions may comprise these active ingredients in prolonged release matrix. In one embodiment, the pharmaceutical composition does not comprise povidone as a binder.

In a further embodiment the present invention relates to the use of povidone for manufacturing a prolonged release pharmaceutical composition comprising oxycodone or a pharmaceutically acceptable salt thereof and naloxone or a pharmaceutically acceptable salt thereof, wherein the pharmaceutical composition after storage under stressed conditions has less than 4 % of total substances related to oxycodone or a pharmaceutically acceptable salt or derivative thereof and/or related to naloxone or a pharmaceutically acceptable salt or derivative thereof.. In some embodiments the composition has equal to or more than 0.05% and preferably equal to or more than 0.1% of total substances related to oxycodone or a pharmaceutically acceptable salt or derivative thereof and/or related to naloxone or a pharmaceutically acceptable salt or derivative thereof.

In yet another embodiment the present invention relates to the use of povidone for manufacturing a prolonged release pharmaceutical composition comprising oxycodone or a pharmaceutically acceptable salt thereof and naloxone or a pharmaceutically acceptable salt thereof, wherein the pharmaceutical composition after storage under stressed conditions releases the pharmaceutically active agents with substantially the same release rate as before subjecting the pharmaceutical composition to stressed conditions.

In these embodiments the pharmaceutical compositions may comprise oxycodone or a pharmaceutically acceptable salt thereof in an amount equivalent to 10 to 160 mg, preferably 16 to 160 mg of oxycodone HCl and naloxone or a pharmaceutically acceptable salt thereof in an amount equivalent to 5 to 80 mg, preferably 8 to 80 mg of naloxone HCl.

These compositions may preferably comprise the active ingredients in 2:1 ratio by weight.

The active ingredients may preferably be oxycodone HCl and naloxone HCl. The pharmaceutical compositions may comprise these active ingredients in prolonged release matrix.

The invention further relates to a method of treating pain in a human or animal being by administering the pharmatceutical compositions described herein. The invention further relates to methods of treating Chronic Obstructive Pulmonary Disorder, Restless Leg, Chrohn's disease, constipation or urinary retention in a human or animal being by administering the pharmatceutical compositions described herein. Treatment of constipation or urinary retention may be particularly considered for patient which due to constipation or urinary retention being induced by opioid therapy would otherwise have to discontinue therapy.

The invention also relates to the aforementioned pharmaceutical compositions, usages and methods where the pharmaceutical compositions comprise at least oxycodone or a pharmaceutically acceptable salt or derivative thereof or naloxone or a pharmaceutically acceptable salt or derivative thereof

### FIGURES

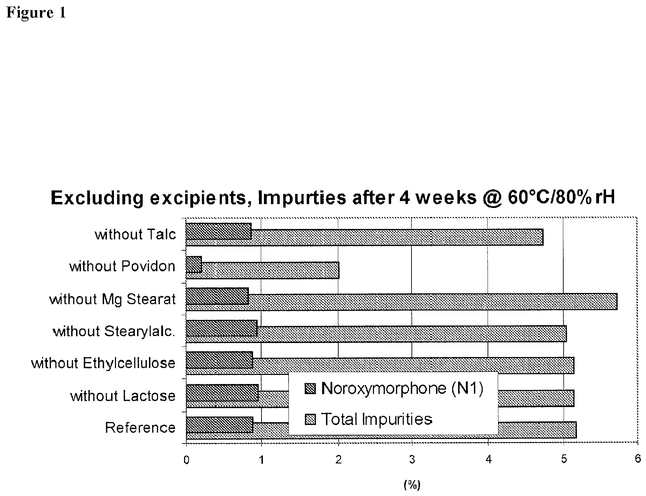

Figure 1 depicts the influence of excipients on impurities.

### DETAILED DESCRIPTION OF THE INVENTION

The present invention as illustratively described in the following may suitably be practiced in the absence of any element or elements, limitation or limitations, not specifically disclosed herein.

The present invention will be described with respect to particular embodiments and with reference to certain figures but the invention is not limited thereto but only by the claims. Terms as set forth hereinafter are generally to be understood in their common sense unless indicated otherwise.

Where the term "comprising" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group which preferably consists only of these embodiments.

Where an indefinite or definite article is used when referring to a singular noun, e.g. "a", "an" or "the", this includes a plural of that noun unless something else is specifically stated.

In the context of the present invention the terms "about" or "approximately" denote an interval of accuracy that the person skilled in the art will understand to still ensure the technical effect of the feature in question. The term typically indicates deviation from the indicated numerical value of ±10%, and preferably of ±5%.

The term "*in vitro* release" and its grammatical variations as well as similar expression refers to the release rate by which a pharmaceutically active agent, e.g. oxycodone HCl is released from the pharmaceutical composition when the in vitro release rate is tested by the paddle method according to the European Pharmacopeia as described in as described in the Ph. Eur.4th edition (2002). The paddle speed is typically set at 50 rpm in 900 ml simulated gastric fluid without pepsin (SGFₛₚ) dissolution medium with pH 1.2. Aliquots of the dissolution media are withdrawn at the respective time points and analysed by HPLC with a MerckLiCrospher 60, RP select B, 5µm, 125x4 mm column, eluted with 75 mM KCl buffer with 15% (v/v) Methanol (pH 2.0) and detected at 230 nm. It is specifically indicated if in the context of the present invention *in vitro* release rates are determined using a different test method (such as SGFₛₚ with 40% (v/v) of ethanol).

The term "Simulated Gastric Fluid, pH 1.2" refers to 0.1 N HCl, pH 1.2 plus 2 g sodium chloride per litre.

In the context of the present invention, the terms "immediate release" or "conventional release" refer to pharmaceutical compositions showing a release of the active substance(s) which is not deliberately modified by a special formulation design and/or manufacturing methods. For oral dosage forms this means that the dissolution profile of the active substance(s) depends essentially on its (theirs) intrinsic properties. Typically, the terms "immediate release" or "conventional release" refer to pharmaceutical compositions which release *in vitro* >75% (by weight) of the pharmaceutically active agent(s) at 45 min.
In the context of the present, the terms "prolonged release" and "controlled release" are used interchangeably and refer to pharmaceutical compositions showing a slower release of the active agent(s) than that of a conventional release pharmaceutical composition administered by the same route. Prolonged or controlled release is achieved by a special formulation design and/or manufacturing method. Typically, the terms "prolonged release" and "controlled release refer to pharmaceutical compositions which release *in vitro* ≤75% (by weight) of the pharmaceutically active agent at 45 min.

Prolonged release properties may be obtained by different means such as by a coating which is then designated as a prolonged release coating, a matrix which is then designated by as a prolonged release matrix or e.g. by an osmotic structure of the pharmaceutical composition.

In order to obtain "prolonged or controlled release" properties, one typically uses materials which are known to prolong the release from a dosage form comprising e.g. a prolonged release matrix and/or prolonged release coating. Typical examples of such "prolonged or controlled release materials" are hydrophobic polymers such as ethyl cellulose, hydrophilic polymers such as hydroxypropyl cellulose and the like. The nature of the "prolonged or controlled release material" may depend on whether the release properties are attained by a "prolonged release matrix" or a "prolonged release coating". The term "prolonged release materials" thus describes both types of materials. The term "prolonged release matrix material" indicates that a material is used for obtaining a prolonged release matrix. Likewise, the term "prolonged release coating material" indicate that a material is used for obtaining a prolonged release coating.

The terms "prolonged release matrix formulation" or "controlled release matrix formulation" refer to a pharmaceutical composition including at least one prolonged release material or controlled release material, and at least one oxycodone and naloxone or the pharmaceutically acceptable salts or derivatives thereof. The terms "prolonged release material" and "controlled release material" can be used interchangeably. In a "prolonged release matrix formulation" or "controlled release matrix formulation", the "prolonged release material" or "controlled release material" are combined with the pharmaceutically active agents to form a mixture from which the pharmaceutically active agent is released over prolonged periods of time, such as e.g. 8, 10, 12, 14, 16, 18, 20, 22 or 24 hours.

It is to be understood that a material will be considered to act as prolonged or controlled release material if the dissolution profile of the pharmaceutically active agent(s) is slowed down compared to an immediate or conventional release formulation. If a prolonged or controlled release material can be used for manufacturing a prolonged or controlled release matrix, it will be considered as a prolonged or controlled release matrix material.

Pharmaceutically acceptable excipients which are used to adjust an already prolonged or controlled release to a specific profile are not necessarily considered to be prolonged or controlled release materials.

It is to be understood that a prolonged release matrix or a controlled release matrix does not necessarily consist only of the pharmaceutically active agent(s) and the prolonged or controlled release material. The prolonged or controlled release matrix may comprise in addition pharmaceutically acceptable excipients such as fillers, lubricants, glidants, etc.

The terms "prolonged release coating formulation" or "controlled release coating formulation" refer to a pharmaceutical composition including at least one prolonged release material or controlled release material, and at least one oxycodone and naloxone or the pharmaceutically acceptable salts or derivatives thereof. The terms "prolonged release material" and "controlled release material" can be used interchangeably. In a "prolonged release coating formulation" or "controlled release coating formulation", the "prolonged release material" or "controlled release material" are disposed on the pharmaceutically active agents to form a diffusion barrier. Other than in prolonged release matrix formulation, the actives are not intimately mixed with the prolonged release material and the prolonged release coating does not form a three dimensional structure within which the actives are distributed. As the term implies, the prolonged release material forms a layer above the actives. The pharmaceutically active agent is released from a prolonged release coating formulation over prolonged periods of time, such as e.g. 8, 10, 12, 14, 16, 18, 20, 22 or 24 hours.

It is to be understood that a material will be considered to act as prolonged or controlled release material if the dissolution profile of the pharmaceutically active agent(s) is slowed down compared to an immediate or conventional release formulation. If a prolonged or controlled release material can be used for manufacturing a prolonged or controlled release coating, it will be considered as a prolonged or controlled release coating material.

Pharmaceutically acceptable excipients which are used to adjust an already prolonged or controlled release to a specific profile are not necessarily considered to be prolonged or controlled release materials.

When it is mentioned that a prolonged release coating is disposed on pharmaceutically active agents, this is not to be construed as meaning that such a coating will necessarily be directly layered on such active pharmaceutically agents. Of course, if pharmaceutically active agents are layered on a carries such as nu-pareil beads, the coating may be disposed directly thereon. However, the pharmaceutically active agents may also be first embedded in a polymer layer or e.g. a prolonged release matrix. Subsequently the prolonged release coating may be disposed on e.g. granules which comprise a prolonged release matrix or on tablets which are made from such granules by compression for example.

A pharmaceutical composition with a controlled or prolonged release coating may be obtained by combining the pharmaceutically active agents with a carrier such as nonpareil beads and disposing a prolonged release coating on said combinations. Such coating may be made from polymers such cellulose ethers with ethyl cellulose being preferred, acrylic resins, other polymers and mixtures thereof. Such controlled or prolonged release coatings may comprise additional excipients such as pore-formers, binders and the like.

It is further to be understood, that the term "prolonged release matrix formulation" or "controlled release matrix formulation" does not exclude pharmaceutical compositions with a prolonged or controlled release matrix and an additional prolonged or controlled release coating being disposed on the matrix. Likewise the term "prolonged release coating formulation" or "controlled release coating formulation" does not exclude pharmaceutical compositions with a prolonged or controlled release coating which is disposed on prolonged release matrix or a controlled release matrix.

In all of its embodiments, the invention is preferably concerned with prolonged release matrix formulations. These formulations may in some embodiments not comprise a prolonged release coating.

The terms "prolonged release dosage form" and "controlled release dosage form" can be used interchangeably and refer to the administration form of a pharmaceutical composition of the present invention comprising the at least one pharmaceutically active agent in prolonged release form as e.g. in form of a "prolonged release matrix formulation", in the form of a "prolonged release coating formulation, combinations thereof or in other prolonged release formulations such as osmotic formulations. The terms "prolonged release matrix formulation" and "prolonged release dosage form" can be used interchangeably if the prolonged release dosage form consists essentially of the prolonged release matrix formulation. This means that a prolonged release dosage form can comprise in addition to the prolonged release matrix e.g. cosmetic coatings and pharmaceutically acceptable excipients such fillers, lubricants, etc.

For some embodiments, the term "prolonged release matrix dosage form" may indicate that the dosage form comprises a prolonged release matrix as the sole structure being responsible for prolonging the release. This, however, does not exclude that the dosage form may comprise an immediate release portion.

For some embodiments, the term "prolonged release coating dosage form" may indicate that the dosage form comprises a prolonged release coating as the sole structure being responsible for prolonging the release. This, however, does not exclude that the dosage form may comprise an immediate release.

The release rates indicated always refer to the formulation such as a monolithic tablet or multi-particulates. The release rates will be chosen such that a pharmaceutical composition can be administered e.g. on a twice a day or once a day basis, i.e. every 12 hours or every 24 hours. Such formulations will thus preferably be therapeutically effective for up to 12 hours or up 14 hours. Typically, the release will occur by diffusion through the prolonged or controlled release matrix and/or coating, erosion of the prolonged or controlled matrix and/or coating or combinations thereof.

Oral solid dosage forms may take the form of tablets, granules, multi-particulates and the like.

The present invention as disclosed herein with respect to all aspects and embodiments is meant to encompass the use of any pharmaceutically acceptable salt or derivative of oxycodone and naloxone. Any embodiment of the invention referring to oxycodone and naloxone is also meant to refer to salts and preferably the hydrochloride salts thereof unless indicated otherwise.

Pharmaceutically acceptable salts include, but are not limited to, inorganic acid salts such as hydrochloride, hydrobromide, sulfate, phosphate and the like; organic acid salts such as formate, acetate, trifluoroacetate, maleate, tartrate and the like; sulfonates such as methanesulfonate, benzenesulfonate, p-toluenesulfonate, and the like; amino acid salts such as arginate, asparginate, glutamate and the like, and metal salts such as sodium salt, potassium salt, cesium salt and the like; alkaline earth metals such as calcium salt, magnesium salt and the like; organic amine salts such as triethylamine salt, pyridine salt, picoline salt, ethanolamine salt, triethanolamine salt, dicyclohexylamine salt, N,N'-dibenzylethylenediamine salt and the like.

Pharmaceutically acceptable derivatives of oxycodone and naloxone include esters thereof as well as modified forms such as glycosylated, pegylated or hesylated forms of oxycodone and naloxone.

If in the following reference is made to a pharmaceutically active agent such as oxycodone, this always also includes the reference to a pharmaceutically acceptable salt or derivative of the free base of this pharmaceutically active agent unless it is specifically indicated that the reference to the pharmaceutically active agent, such as use of the term "oxycodone" should only refer to the free base.

The use of the hydrochloride salts of both oxycodone and naloxone can be preferred.

In a preferred embodiment, the pharmaceutical dosage forms comprise oxycodone or a pharmaceutically acceptable salt or derivative thereof or naloxone or a pharmaceutically acceptable salt or derivative thereof as the sole pharmaceutically active agents.

The pharmaceutical compositions may comprise about 1 to about 160 mg such as about 1 mg, about 5 mg, about 10 mg, about 15 mg, about 16 mg, about 20 mg, about 30 mg, about 40 mg, about 60 mg, about 80 mg, about 100 mg, about 120 mg, about 140 mg or about 160 mg oxycodone hydrochloride or equimolar amounts of any other pharmaceutically acceptable salt or derivative including but not limited to hydrates and solvates or of the free base.

The pharmaceutical compositions may comprise about 0.5 to about 80 mg, such as about 1 mg, about 1.25 mg, about 2 mg, about 2.5 mg, about 3 mg, about 3.5 mg, about 3.75 mg, about 4 mg, about 4.5 mg, about 5 mg, about 6 mg, about 7 mg, about 7.5 mg, about 8 mg, about 9 mg, about 10 mg, about 15 mg, about 20 mg, about 30 mg, about 40 mg, about 50 mg, about 60 mg, about 70 mg or about 80 mg of naloxone hydrochloride or equimolar amounts of any other pharmaceutically acceptable salt, derivative or form including but not limited to hydrates and solvates or of the free base.

In some embodiments, the composition comprises oxycodone or a pharmaceutically acceptable salt or derivative thereof in an amount equivalent to 1 mg of oxycodone HCl and naloxone or a pharmaceutically acceptable salt or derivative thereof in an amount equivalent to 0.5 mg of naloxone HCl, oxycodone or a pharmaceutically acceptable salt or derivative thereof in an amount equivalent to 2.5 mg of oxycodone HCl and naloxone or a pharmaceutically acceptable salt or derivative thereof in an amount equivalent to 1.25 mg of naloxone HCl, oxycodone or a pharmaceutically acceptable salt or derivative thereof in an amount equivalent to 5 mg of oxycodone HCl and naloxone or a pharmaceutically acceptable salt or derivative thereof in an amount equivalent to 2.5 mg of naloxone HCl, oxycodone or a pharmaceutically acceptable salt or derivative thereof in an amount equivalent to 7.5 mg of oxycodone HCl and naloxone or a pharmaceutically acceptable salt or derivative thereof in an amount equivalent to 3.75 mg of naloxone HCl, oxycodone or a pharmaceutically acceptable salt or derivative thereof in an amount equivalent to 10 mg of oxycodone HCl and naloxone or a pharmaceutically acceptable salt or derivative thereof in an amount equivalent to 5 mg of naloxone HCl, oxycodone or a pharmaceutically acceptable salt or derivative thereof in an amount equivalent to 12.5 mg of oxycodone HCl and naloxone or a pharmaceutically acceptable salt or derivative thereof in an amount equivalent to 6.25 mg of naloxone HCl, or oxycodone or a pharmaceutically acceptable salt or derivative thereof in an amount equivalent to 15 mg of oxycodone HCl and naloxone or a pharmaceutically acceptable salt or derivative thereof in an amount equivalent to 7.5 mg of naloxone HCl.

In other embodiments the pharmaceutical composition comprises oxycodone or a pharmaceutically acceptable salt or derivative thereof in an amount equivalent to 10 mg of oxycodone HCl and naloxone or a pharmaceutically acceptable salt or derivative thereof in an amount equivalent to 5 mg of naloxone HCl, oxycodone or a pharmaceutically acceptable salt or derivative thereof in an amount equivalent to 15 mg of oxycodone HCl and naloxone or a pharmaceutically acceptable salt or derivative thereof in an amount equivalent to 7.5 mg of naloxone HCl, oxycodone or a pharmaceutically acceptable salt or derivative thereof in an amount equivalent to 20 mg of oxycodone HCl and naloxone or a pharmaceutically acceptable salt or derivative thereof in an amount equivalent to 10 mg of naloxone HCl, oxycodone or a pharmaceutically acceptable salt or derivative thereof in an amount equivalent to 30 mg of oxycodone HCl and naloxone or a pharmaceutically acceptable salt or derivative thereof in an amount equivalent to 15 mg of naloxone HCl, oxycodone or a pharmaceutically acceptable salt or derivative thereof in an amount equivalent to 40 mg of oxycodone HCl and naloxone or a pharmaceutically acceptable salt or derivative thereof in an amount equivalent to 20 mg of naloxone HCl, oxycodone or a pharmaceutically acceptable salt or derivative thereof in an amount equivalent to 60 mg of oxycodone HCl and naloxone or a pharmaceutically acceptable salt or derivative thereof in an amount equivalent to 30 mg of naloxone HCl, oxycodone or a pharmaceutically acceptable salt or derivative thereof in an amount equivalent to 80 mg of oxycodone HCl and naloxone or a pharmaceutically acceptable salt or derivative thereof in an amount equivalent to 40 mg of naloxone HCl and oxycodone or a pharmaceutically acceptable salt or derivative thereof in an amount equivalent to 160 mg of oxycodone HCl and naloxone or a pharmaceutically acceptable salt or derivative thereof in an amount equivalent to 80 mg of naloxone HCl.

In some embodiments, the prolonged release pharmaceutical compositions in accordance with the invention comprise at least oxycodone or a pharmaceutically acceptable salt or derivative thereof and naloxone or a pharmaceutically acceptable salt or derivative thereof in a manner as described above, wherein the amount of oxycodone or a pharmaceutically acceptable salt or derivative thereof and naloxone or a pharmaceutically acceptable salt or derivative thereof released *in vitro* in 500 ml of Simulated Gastric Fluid, pH 1.2 using the Ph. Eur. paddle method at 50 rpm at 37° C is:

| | |
|---|---|
| at 15 min: | 10 to 30 % by weight of the pharmaceutically active agents, |
| at 2 h: | 40 to 60% by weight of the pharmaceutically active agents, |
| at 10 h: | more than 80% by weight of the pharmaceutically active agents. |

The above release rates may apply to prolonged release pharmaceutical compositions which comprise oxycodone or a pharmaceutically acceptable salt or derivative thereof in an amount equivalent to of less than 10 mg oxycodone HCl and naloxone or a pharmaceutically acceptable salt thereof in an amount equivalent to of less than 5 mg naloxone HCl. Further, the above release rates may apply to prolonged release pharmaceutical compositions which comprise oxycodone or a pharmaceutically acceptable salt or derivative thereof in an amount equivalent to of equal to or more than 40 mg oxycodone HCl and naloxone or a pharmaceutically acceptable salt thereof in an amount equivalent to of equal to or more than 20 mg naloxone HCl.

The prolonged release pharmaceutical composition may comprise these actives in the above indicated amounts and weight ratio of about 2:1. The pharmaceutically active agents may preferably be oxycodone HCl and naloxone HCl being preferred.

Preferably, the amount of the pharmaceutically active agents released *in vitro* in 900 ml of Simulated Gastric Fluid, pH 1.2 using the Ph. Eur. paddle method at 50 rpm at 37° C is:

| | |
|---|---|
| at 15 min: | 10 to 30% by weight of the pharmaceutically active agents, |
| at 1 h: | 25 to 45% by weight of the pharmaceutically active agents, |
| at 2 h: | 40 to 60% by weight of the pharmaceutically active agents, |
| at 4 h: | 55 to 75% by weight of the pharmaceutically active agents, |
| at 7 h: | 70 to 90% by weight of the pharmaceutically active agents, |
| at 10 h: | more than 80% by weight of the pharmaceutically active agents. |

The above release rates may apply to prolonged release pharmaceutical compositions which comprise oxycodone or a pharmaceutically acceptable salt or derivative thereof in an amount equivalent to of less than 10 mg oxycodone HCl and naloxone or a pharmaceutically acceptable salt thereof in an amount equivalent to of less than 5 mg naloxone HCl. Further, the above release rates may apply to prolonged release pharmaceutical compositions which comprise oxycodone or a pharmaceutically acceptable salt or derivative thereof in an amount equivalent to of equal to or more than 40 mg oxycodone HCl and naloxone or a pharmaceutically acceptable salt thereof in an amount equivalent to of equal to or more than 20 mg naloxone HCl. The prolonged release pharmaceutical composition may comprise these actives in the above indicated amounts and weight ratio of about 2:1. The pharmaceutically active agents may preferably be oxycodone HCl and naloxone HCl being preferred.

More preferably, the amount of the pharmaceutically active agents released *in vitro* in 900 ml of Simulated Gastric Fluid, pH 1.2 using the Ph. Eur. paddle method at 50 rpm at 37° C is:

| | |
|---|---|
| at 15 min: | 15 to 25% by weight of the pharmaceutically active agents, |
| at 1 h: | 30 to 40% by weight of the pharmaceutically active agents, |
| at 2 h: | 45 to 55% by weight of the pharmaceutically active agents, |
| at 4 h: | 60 to 70% by weight of the pharmaceutically active agents, |
| at 7 h: | 75 to 85% by weight of the pharmaceutically active agents, |
| at 10 h: | more than 80% by weight of the pharmaceutically active agents. |

The above release rates may apply to prolonged release pharmaceutical compositions which comprise oxycodone or a pharmaceutically acceptable salt or derivative thereof in an amount equivalent to of less than 10 mg oxycodone HCl and naloxone or a pharmaceutically acceptable salt thereof in an amount equivalent to of less than 5 mg naloxone HCl. Further, the above release rates may apply to prolonged release pharmaceutical compositions which comprise oxycodone or a pharmaceutically acceptable salt or derivative thereof in an amount equivalent to of equal to or more than 40 mg oxycodone HCl and naloxone or a pharmaceutically acceptable salt thereof in an amount equivalent to of equal to or more than 20 mg naloxone HCl. The prolonged release pharmaceutical composition may comprise these actives in the above indicated amounts and weight ratio of about 2:1. The pharmaceutically active agents may preferably be oxycodone HCl and naloxone HCl being preferred.

In some embodiments, the prolonged release pharmaceutical compositions in accordance with the invention comprise at least oxycodone or a pharmaceutically acceptable salt or derivative thereof and naloxone or a pharmaceutically acceptable salt or derivative thereof in a manner as described above, wherein the amount of oxycodone or a pharmaceutically acceptable salt or derivative thereof and naloxone or a pharmaceutically acceptable salt or derivative thereof released *in vitro* in 900 ml of Simulated Gastric Fluid, pH 1.2 using the Ph. Eur. paddle method at 50 rpm at 37° C is:

| | |
|---|---|
| at 15 min: | 10 to 30 % by weight of the pharmaceutically active agents, |
| at 2 h: | 45 to 65% by weight of the pharmaceutically active agents, |
| at 10 h: | more than 80% by weight of the pharmaceutically active agents. |

The above release rates may apply to prolonged release pharmaceutical compositions which comprise oxycodone or a pharmaceutically acceptable salt or derivative thereof in an amount equivalent to of equal to or more than 10 mg oxycodone HCl and naloxone or a pharmaceutically acceptable salt thereof in an amount equivalent to of equal to or more than 5 mg naloxone HCl. Further, the above release rates may apply to prolonged release pharmaceutical compositions which comprise oxycodone or a pharmaceutically acceptable salt or derivative thereof in an amount equivalent to of less than 40 mg oxycodone HCl and naloxone or a pharmaceutically acceptable salt thereof in an amount equivalent to of less than 20 mg naloxone HCl. The prolonged release pharmaceutical composition may comprise these actives in the above indicated amounts and weight ratio of about 2:1. The pharmaceutically active agents may preferably be oxycodone HCl and naloxone HCl being preferred.

Preferably, the amount of the pharmaceutically active agents released *in vitro* in 900 ml of Simulated Gastric Fluid, pH 1.2 using the Ph. Eur. paddle method at 50 rpm at 37° C is:

| | |
|---|---|
| at 15 min: | 10 to 30% by weight of the pharmaceutically active agents, |
| at 1 h: | 30 to 50% by weight of the pharmaceutically active agents, |
| at 2 h: | 45 to 65% by weight of the pharmaceutically active agents, |
| at 4 h: | 65 to 85% by weight of the pharmaceutically active agents, |
| at 7 h: | 75 to 95% by weight of the pharmaceutically active agents, |
| at 10 h: | more than 80% by weight of the pharmaceutically active agents. |

The above release rates may apply to prolonged release pharmaceutical compositions which comprise oxycodone or a pharmaceutically acceptable salt or derivative thereof in an amount equivalent to of equal to or more than 10 mg oxycodone HCl and naloxone or a pharmaceutically acceptable salt thereof in an amount equivalent to of equal to or more than 5 mg naloxone HCl. Further, the above release rates may apply to prolonged release pharmaceutical compositions which comprise oxycodone or a pharmaceutically acceptable salt or derivative thereof in an amount equivalent to of less than 40 mg oxycodone HCl and naloxone or a pharmaceutically acceptable salt thereof in an amount equivalent to of less than 20 mg naloxone HCl. The prolonged release pharmaceutical composition may comprise these actives in the above indicated amounts and weight ratio of about 2:1. The pharmaceutically active agents may preferably be oxycodone HCl and naloxone HCl being preferred.

More preferably, the amount of the pharmaceutically active agents released *in vitro* in 900 ml of Simulated Gastric Fluid, pH 1.2 using the Ph. Eur. paddle method at 50 rpm at 37° C is:

| | |
|---|---|
| at 15 min: | 15 to 25% by weight of the pharmaceutically active agents, |
| at 1 h: | 35 to 45% by weight of the pharmaceutically active agents, |
| at 2 h: | 50 to 60% by weight of the pharmaceutically active agents, |
| at 4 h: | 70 to 80% by weight of the pharmaceutically active agents, |
| at 7 h: | 80 to 90% by weight of the pharmaceutically active agents, |
| at 10 h: | more than 80% by weight of the pharmaceutically active agents. |

The above release rates may apply to prolonged release pharmaceutical compositions which comprise oxycodone or a pharmaceutically acceptable salt or derivative thereof in an amount equivalent to of equal to or more than 10 mg oxycodone HCl and naloxone or a pharmaceutically acceptable salt thereof in an amount equivalent to of equal to or more than 5 mg naloxone HCl. Further, the above release rates may apply to prolonged release pharmaceutical compositions which comprise oxycodone or a pharmaceutically acceptable salt or derivative thereof in an amount equivalent to of less than 40 mg oxycodone HCl and naloxone or a pharmaceutically acceptable salt thereof in an amount equivalent to of less than 20 mg naloxone HCl. The prolonged release pharmaceutical composition may comprise these actives in the above indicated amounts and weight ratio of about 2:1. The pharmaceutically active agents may preferably be oxycodone HCl and naloxone HCl being preferred.

As mentioned above the present invention aims at providing pharmaceutical compositions which after storage under stressed conditions release the pharmaceutically active agents with substantially the same release rate as before subjecting the pharmaceutical composition to stressed conditions.

Storage under stressed conditions in the context of the present invention means that a pharmaceutical composition is subjected to increased temperature and/or relative humidity (RH) for prolonged periods of time. For example, typical stressed conditions refer to storage over at least one, two, three, four, five or six months at 25°C and 60% RH, at 30°C and 65% RH, at 30°C and 75% RH, at 40°C and 60% RH or at 40°C and 75% RH. These conditions have been used to some extent in the examples described herein. Storage under stressed conditions has e.g. been performed for 3 months at 25°C and 60% RH, for 3 months at 30°C and 65% RH, for 3 months at 40°C and 60% RH, for 6 months at 25°C and 60% RH, for 3 months at 30°C and 75% RH, or for 3 months at 40°C and 75% RH.

Such stressed storage conditions are used to determine whether a pharmaceutical composition has a shelf life sufficient for long time storage under conditions as they are common in patients' households without negative effects on its safety and efficacy. Such negative effects may include that the in-vitro release rates change over time so that the efficacy of the composition is affected as different amounts of actives are released after administration. Similarly, negative effects may also result from degradation of the pharmaceutically active agents which may either decrease the overall amount of functional pharmaceutically active agent or lead to formation of toxic by-products.

If changes in the in vitro release profile or with respect to the amount of the active agent(s) of a pharmaceutical composition are observed after storage under stressed conditions, this may be indicative of stability problems. If such changes are not observed, this means vice versa that the pharmaceutical composition is storage stable.

The term "substantially the same release rate" refers to the situation where the in vitro release rate for a pharmaceutical composition which has been subjected to stressed conditions is compared to a reference composition. The reference composition is an identical pharmaceutical composition which, however, has not been subjected to stressed conditions. If the in vitro release profile of the composition subjected to stressed conditions does not deviate by more than about 25%, preferably by no more than about 20%, more preferably by no more than 15% and even more preferably by no more than about 10% or 5% from the in vitro release profile of the reference composition, the in-vitro release rate is considered to be substantially the same.

In one embodiment, the present invention thus aims at providing pharmaceutical compositions which after storage for 3, 6, 9 or 12 months at 25°C and 60% RH release the pharmaceutically active agents with substantially the same release rate as before subjecting the pharmaceutical composition to stressed conditions.

In another embodiment, the present invention thus aims at providing pharmaceutical compositions which after storage for 3, 6, 9 or 12 months at 30°C and 65% RH release the pharmaceutically active agents with substantially the same release rate as before subjecting the pharmaceutical composition to stressed conditions.

In another embodiment, the present invention thus aims at providing pharmaceutical compositions which after storage for 3, 6, 9 or 12 months at 30°C and 75% RH release the pharmaceutically active agents with substantially the same release rate as before subjecting the pharmaceutical composition to stressed conditions.

In yet another embodiment, the present invention thus aims at providing pharmaceutical compositions which after storage for 3, 6, 9 or 12 months at 40°C and 60% RH release the pharmaceutically active agents with substantially the same release rate as before subjecting the pharmaceutical composition to stressed conditions.

In yet another embodiment, the present invention thus aims at providing pharmaceutical compositions which after storage for 3, 6, 9 or 12 months at 40°C and 75% RH release the pharmaceutically active agents with substantially the same release rate as before subjecting the pharmaceutical composition to stressed conditions.

In a further aspect the present invention aims at providing pharmaceutical compositions which after storage under stressed conditions have less than 4 % of total substances related to oxycodone or a pharmaceutically acceptable salt or derivative thereof and/or related to naloxone or a pharmaceutically acceptable salt or derivative thereof. In some embodiments the composition has equal to or more than 0.05% and preferably equal to or more than 0.1% of total substances related to oxycodone or a pharmaceutically acceptable salt or derivative thereof and/or related to naloxone or a pharmaceutically acceptable salt or derivative thereof.

In a further aspect the present invention aims at providing pharmaceutical compositions which after storage under stressed conditions have less than 0.5 % of known substances or a known substance related to oxycodone or a pharmaceutically acceptable salt or derivative thereof and less than 0.5% of known substances related to naloxone or a pharmaceutically acceptable salt or derivative thereof. In some embodiments the composition has equal to or more than 0.01% and preferably equal to or more than 0.07% of known substances or a known substance related to oxycodone or a pharmaceutically acceptable salt or derivative thereof and/or related to naloxone or a pharmaceutically acceptable salt or derivative thereof. These values preferably relate to the known related substance noroxymorphone.

The term "total oxycodone and naloxone related substances" or the like refers to substances that arise from chemical reactions of oxycodon or naloxone, pharmaceutically acceptable salts and derivatives thereof such as e.g. degradation. These substances can be distinguished as known oxycodone related substances where the identity of the substance and its origin is known, as known naloxone related substances where the identity of the substance and its origin is known, and as unkown substances. For unknown substances, their identiy is not known. However, it is assumed that they arise from oxycodone and/or naloxone, pharmaceutically acceptable salts and derivatives thereof. It is to be understood that the term "total oxycodone and naloxone related substances" includes the sum of known oxycodone related substances, known naloxone related substances and unknown substances.

Terms like "less than 4 % of total substances related to oxycodone and naloxone, or to pharmaceutically acceptable salts or derivatives thereof" or "less than 3.5 % of total substances related to oxycodone and naloxone or to pharmaceutically acceptable salts or derivatives thereof' etc. indicate that the amount of total substances as described in the preceding paragraph is less than e.g. 4% or 3.5% by weight based on the total amount of the active ingredient (i.e. oxycodone or naloxone), or a pharmaceutically acceptable salt or derivative thereof which is present in the pharmaceutical composition in the lower amount. Thus, if a pharmaceutical composition comprises oxycodone HCl and naloxone HCl in 2:1 weight ratio by weight, the amount of total substances is calculated from the sum of known oxycodone HCl related substances, known naloxone HCl related substances and unknown substances which is then referenced to the amount of naloxone HCl.

"Known oxycodone related substances" include e.g. 6-a oxycodol, noroxycodon, 14-hydroxycodeinon, oxycodon-N-oxid and hydrocodon base. "Known naloxone related substances" include noroxymorphon, 10a-hydroxynaloxon, 7,8-didehydronaloxon, pseudonaloxon, 3-o-allylnaloxon.

Terms like "less than 4 % of known substances related to oxycodone, or to pharmaceutically acceptable salts or derivatives thereof" or "less than 3.5 % of known substances related to oxycodone, or to pharmaceutically acceptable salts or derivatives thereof' etc. indicate that the amount of known oxycodone related substances is less than e.g. 4% or 3.5% per known oxycodone related substance by weight based on the total amount of oxycodone, or a pharmaceutically acceptable salt or derivative thereof in the composition.

Terms like "less than 4 % of known substances related to naloxone, or to pharmaceutically acceptable salts or derivatives thereof' or "less than 3.5 % of known substances related to naloxone, or to pharmaceutically acceptable salts or derivatives thereof' etc. indicate that the amount of known naloxone related substances is less than e.g. 4% or 3.5% per known naloxone related substance by weight based on the total amount of naloxone, or a pharmaceutically acceptable salt or derivative thereof in the composition.

The term "substance related to oxycodone, or to pharmaceutically acceptable salts or derivatives thereof' relates to an entity that is different from the oxycodone, or the pharmaceutically acceptable salts or derivatives thereof which are used as pharmaceutically active agent in the composition.

The term "substance related to naloxone, or to pharmaceutically acceptable salts or derivatives thereof' relates to an entity that is different from the naloxone, or the pharmaceutically acceptable salts or derivatives thereof which are used as pharmaceutically active agent in the composition.

In order to assess stability, one may subject a pharmaceutical composition to stressed conditions as mentioned above and then determine the amount of total oxycodone and/or naloxone related substances. Determining the amount of of "total oxycodone and/or naloxone related substances" is typically performed by HPLC analysis. For example, one may mill a pharmaceutical composition such as tablet comprising oxycodone HCl and naloxone HCl and extract the active ingredients including related substances and separate them by HPCL using e.g. Hypersil BDS C18, 3 µm 3,0*100 mm column from Thermo Finigan at 210nm. Oxycodone HCl and Naloxone HCl as well as known related substances are identified by using corresponding pure substances as standards. The amount of the substances including the amount of unknown substances is then determined by calculating the area under the respective peaks in the chromatogram.

Stressed storage conditions may be the same as mentioned above. Thus typical stressed conditions may refer to storage over at least one, two, three, four, five or six months at 25°C and 60% RH, at 30°C and 65% RH, at 30°C and 75% RH, at 40°C and 60% RH at 40°C and 75%.

The amount of total substances is preferably less than 4%, preferably less than 3.5%, more preferably less than 3%, even more preferably less than 2% or most preferably less than 1% or even equal to or less than 0.5%. In some embodiments the composition has equal to or more than 0.05% and preferably equal to or more than 0.1% of total substances related to oxycodone or a pharmaceutically acceptable salt or derivative thereof and/or related to naloxone or a pharmaceutically acceptable salt or derivative thereof.

The amount of known oxycodone related substances or known naloxone related substances is less than 0.5%, preferably less than 0.4%, more preferably less than 0.3%, even more preferably less than 0.2% and most preferably less than 0.1%. In some embodiments the composition has equal to or more than 0.01% and preferably equal to or more than 0.07% of known substances related to oxycodone or a pharmaceutically acceptable salt or derivative thereof and/or related to naloxone or a pharmaceutically acceptable salt or derivative thereof. These values preferably relate to the known related substance noroxymorphone.

The pharmaceutical compositions after storage under stressed conditions has less than 0.5 % of single known related substance related to oxycodone or a pharmaceutically acceptable salt or derivative thereof and/or related to naloxone or a pharmaceutically acceptable salt or derivative thereof.In some embodiments the composition has equal to or more than 0.01% and preferably equal to or more than 0.07% of a known substance related to oxycodone or a pharmaceutically acceptable salt or derivative thereof and/or related to naloxone or a pharmaceutically acceptable salt or derivative thereof. These values preferably relate to the known related substance noroxymorphone.

In one embodiment, the present invention thus aims compositions which after storage for 3, 6, 9 or 12 months at 25°C and 60% RH have less than 4 %, less than 3.5%, less than 3%, less than 2% or even less than 1% or even equal to or less than 0.5% of total substances related to oxycodone or a pharmaceutically acceptable salt or derivative thereof and related to naloxone or a pharmaceutically acceptable salt or derivative thereof. The lower limits may be as described above.

In another embodiment, the present invention thus aims at providing pharmaceutical compositions which after storage for 3, 6, 9 or 12 months at 30°C and 65% RH have less than 4%, less than 3.5%, less than 3%, less than 2% or even less than 1% or even equal to or less than 0.5% of total substances related to oxycodone or a pharmaceutically acceptable salt or derivative thereof and related to naloxone or a pharmaceutically acceptable salt or derivative thereof. The lower limits may be as described above.

In another embodiment, the present invention thus aims at providing pharmaceutical compositions which after storage for 3, 6, 9 or 12 months at 30°C and 75% RH have less than 4%, less than 3.5%, less than 3%, less than 2% or even less than 1% or even equal to or less than 0.5% of total substances related to oxycodone or a pharmaceutically acceptable salt or derivative thereof and related to naloxone or a pharmaceutically acceptable salt or derivative thereof. The lower limits may be as described above.

In yet another embodiment, the present invention thus aims at providing pharmaceutical compositions which after storage for 3, 6, 9 or 12 months at 40°C and 60% RH have less than 4 %, less than 3.5%, less than 3%, less than 2% or even less than 1% or even equal to or less than 0.5% of total substances related to oxycodone or a pharmaceutically acceptable salt or derivative thereof and related to naloxone or a pharmaceutically acceptable salt or derivative thereof. The lower limits may be as described above.

In yet another embodiment, the present invention thus aims at providing pharmaceutical compositions which after storage for 3, 6, 9 or 12 months at 40°C and 75% RH have less than 4 %, less than 3.5%, less than 3%, less than 2% or even less than 1% or even equal to or less than 0.5% of total substances related to oxycodone or a pharmaceutically acceptable salt or derivative thereof and related to naloxone or a pharmaceutically acceptable salt or derivative thereof. The lower limits may be as described above.

In one embodiment, the present invention thus aims compositions which after storage for 3, 6, 9, or 12 months at 25°C and 60% RH have less than 0.5%, less than 0.4%, less than 0.3%, less than 0.2% or less than 0.1% of known substances or a kown substance related to oxycodone or a pharmaceutically acceptable salt or derivative thereof and less than 0.5%, less than 0.4%, less than 0.3%, less than 0.2% or less than 0.1% of known substances or a known substance related to naloxone or a pharmaceutically acceptable salt or derivative thereof. The lower limits may be as described above. A preferred known oxycodone related substance may be noroxymorphone.

In another embodiment, the present invention thus aims at providing pharmaceutical compositions which after storage for 3, 6, 9, or 12 months at 30°C and 65% RH have and less than 0.5%, less than 0.4%, less than 0.3%, less than 0.2% or less than 0.1% of known substances or a known substance related to oxycodone or a pharmaceutically acceptable salt or derivative thereof and less than 0.5%, less than 0.4%, less than 0.3%, less than 0.2% or less than 0.1% of known substances or a known substance related to naloxone or a pharmaceutically acceptable salt or derivative thereof. The lower limits may be as described above. A preferred known oxycodone related substance may be noroxymorphone.

In another embodiment, the present invention thus aims at providing pharmaceutical compositions which after storage for 3, 6, 9, or 12 months at 30°C and 75% RH have and less than 0.5%, less than 0.4%, less than 0.3%, less than 0.2% or less than 0.1% of known substances or a known substance related to oxycodone or a pharmaceutically acceptable salt or derivative thereof and less than 0.5%, less than 0.4%, less than 0.3%, less than 0.2% or less than 0.1% of known substances or a known substance related to naloxone or a pharmaceutically acceptable salt or derivative thereof. The lower limits may be as described above. A preferred known oxycodone related substance may be noroxymorphone.

In another embodiment, the present invention thus aims at providing pharmaceutical compositions which after storage for 3, 6, 9, or 12 months at 40°C and 60% RH have and less than 0.5%, less than 0.4%, less than 0.3%, less than 0.2% or less than 0.1% of known substances or a known substance related to oxycodone or a pharmaceutically acceptable salt or derivative thereof and less than 0.5%, less than 0.4%, less than 0.3%, less than 0.2% or less than 0.1% of known substances or a known substance related to naloxone or a pharmaceutically acceptable salt or derivative thereof. The lower limits may be as described above. A preferred known oxycodone related substance may be noroxymorphone.

In another embodiment, the present invention thus aims at providing pharmaceutical compositions which after storage for 3, 6, 9, or 12 months at 40°C and 75% RH have and less than 0.5%, less than 0.4%, less than 0.3%, less than 0.2% or less than 0.1% of known substances or a known substance related to oxycodone or a pharmaceutically acceptable salt or derivative thereof and less than 0.5%, less than 0.4%, less than 0.3%, less than 0.2% or less than 0.1% of known substances or a known substance related to naloxone or a pharmaceutically acceptable salt or derivative thereof. The lower limits may be as described above. A preferred known oxycodone related substance may be noroxymorphone.

In yet another embodiment, the present invention thus aims at providing pharmaceutical compositions which after storage for 3, 6, 9, or 12 months at 40°C and 60% RH have less than 0.5%, less than 0.4%, less than 0.3%, less than 0.2% or less than 0.1% of known substances related to oxycodone or a pharmaceutically acceptable salt or derivative thereof and less than 0.5%, less than 0.4%, less than 0.3%, less than 0.2% or less than 0.1% of a single known substance related to oxycodone or a pharmaceutically acceptable salt or derivative thereof. The lower limits may be as described above. A preferred known oxycodone related substance may be noroxymorphone.

In yet another embodiment, the present invention thus aims at providing pharmaceutical compositions which after storage for 3, 6, 9, or 12 months at 40°C and 75% RH have less than 0.5%, less than 0.4%, less than 0.3%, less than 0.2% or less than 0.1% of known substances related to oxycodone or a pharmaceutically acceptable salt or derivative thereof and less than 0.5%, less than 0.4%, less than 0.3%, less than 0.2% or less than 0.1% of a single known substance related to oxycodone or a pharmaceutically acceptable salt or derivative thereof. The lower limits may be as described above. A preferred known oxycodone related substance may be noroxymorphone.

It will be described below how prolonged release pharmaceutical compositions of oxycodone and naloxone being stable can be manufactured. It will be apparent from this description that selection of e.g. binders may be a means to positively influence stability. Heat treatment may also improve physical stability such as robustness and hardness and also positively influence chemical stability.

Heat treatment may be performed such that the hardness/breaking strength of the pharmaceutical composition is increased for the heat treated versus the non heat treated composition. By heat treating pharmaceutical compositions in accordance with the invention, one may thus obtain compositions with improved hardness/breaking strength proportional to the tablet weight, size and shape. Heat treatment may improve physical stability such as robustness and hardness and also positively influence chemical stability.

After heat treatment for relatively short periods of time (e.g. 30 min at 55°C) the *in vitro* release rate may not change any further upon further heat treatment, i.e. remains substantially the same. It is further observed that such heat treated compositions when being subjected to stressed conditions may have substantially the same *in vitro* release rate as the same heat treated composition which has not been subjected to stressed conditions.

The term "heat treatment" refers to a thermal treatment under either or both increased temperature for a prolonged period of time. Typically, heat treatment takes place at a temperature in the range of about 30°C to about 95°C and for a time in the range of about 10 min to about 3 hours. Typically heat treatment conditions may thus be treatment for at least about 15 min, at least about 30 min, at least about 45 min, at least about 60 min, at least about 75 min, at least about 90 min, at least about 120 min, at least about 150 min, at least about 180 min or at least about 240 min at about at least 30°C, at about at least 40°C, at about at least 50°C, at about at least 60°C or at about at least 80°C at ambient humidity.

Heat treatment can be performed in a convection oven, in an open oven, under vacuum, in the coating drum using conventional heat, microwave and any other sources of heat. Heat treatment in a coating drum can be preferred

As mentioned above, pharmaceutical compositions in accordance with the invention may comprise a prolonged release coating and/or prolonged release matrix. Use of a prolonged release matrix can be preferred. For manufacturing of a prolonged release matrix, one may use prolonged release matrix materials.

Such materials may be hydrophilic and/or hydrophobic materials such as gums, cellulose ethers or acrylic polymers.

Prolonged materials may also include fatty acids, fatty alcohols, glyceryl esters of fatty acids, polyethylene glycols, oils and waxes. Fatty acids and fatty alcohols preferably are those with a C₁₀ to C₃₀ chain, preferably with a C₁₂ to C₂₄ chain and more preferably with a C₁₄ to C₂₀ chain or a C₁₆ to C₂₀ chain. Materials such as stearyl alcohol, cetostearyl alcohol, cetyl alcohol, myristyl alcohol and polyalkylene glycols may be preferred. Waxes may be selected from natural and synthetic waxes such as beeswax, carnauba wax. Oils may include mineral oils or vegetable oils such as castor oil or hydrogenated castor oil.

The prolonged release matrix materials which may be considered in the context of the present invention may also be selected from cellulose ethers.

The term "cellulose ethers" comprises cellulose-derived polymers derivatized with at least alkyl and/or hydroxyalkyl groups which may be hydrophilic or hydrophobic.

For example, the prolonged release matrix material may be a hydrophilic hydroxy alkyl cellulose such as a hydroxy (C1 - C6) alkyl celluloses such as hydroxypropyl cellulose, hydroxypropylmethyl cellulose and particularly preferably hydroxyethyl cellulose.

Examples of hydrophobic cellulose ethers include e.g. ethyl cellulose. The use of ethyl cellulose may be preferred. Hydrophobic cellulose ethers such as ethyl cellulose may be particularly suitable for imparting alcohol resistance to pharmaceutical compositions.

A particularly suitable material for prolonged release matrix formulations in accordance with the present invention may be selected from the group of acrylic resins. Such acrylic resins may be made from (meth) acrylic acid (co) polymers.

There are various types of (meth)acrylic acid (co)polymers available which may be characterised according to the nature of their residues such as neutral (meth)acrylic acid (co)polymers, (meth)acrylic acid (co)polymers with anionic residues or (meth)acrylic acid ester copolymers with cationic residues.

Neutral (meth)acrylic acid (co)polymers include polymers having 95 to 100% by weight of polymerised monomers having neutral residues. Monomers with neutral residues can be C₁-C₄ alkyl esters of acrylic or methacrylic acid such as methylmethacrylate, ethylmethacrylate, butylmethacrylate, methylacrylate, ethylacrylate and butylacrylate. For example, neutral (meth)acrylic acid (co)polymers may comprise 20 to 40 % by weight ethylacrylate and 60 to 80 % by weight methylmethacrylate. Such polymers are e.g. available under the trade name Eudragit® NE which is a copolymer of 30 % by weight ethylacrylate and 70 % by weight methylmethacrylate. This polymer is usually provided in the form of a 30 % or 40% aqueous dispersion (Eudragit® NE 30 D, Eudragit® NE 40 D or Eudragit® NM 30 D).

(Meth)acrylic acid (co)polymers with functional anionic residues may be (meth)acrylic acid (co)polymers having 25 to 95 % by weight of radically polymerised C₁ to C₄ alkyl esters of acrylic or methacrylic acid and 5 to 75 % by weight of methacrylate monomers with an anionic group in the alkyl residue. C₁ to C₄ alkyl esters of acrylic or methacrylic acid are again methylmethacrylate, ethyl methacrylate, butylmethacrylate, methylacrylate, ethylacrylate and butylacrylate. A (meth)acrylate monomer with an anionic group in the alkyl residue may be for example acrylic acid and preferably methacrylic acid. Such methacrylic acid copolymers with an anionic functional group may comprise e.g. 40 to 60 % by weight methacrylic acid and 60 to 40 % by weight methylmethacrylate or 60 to 40 % by weight ethyl acrylate. These types of polymers are available as Eudragit® L 100 / Eudragit® L 12.5 or Eudragit® L 100-55 / Eudragit® L 30 D-55, respectively.

For example, Eudragit® L 100 is a copolymer of 50 % by weight methylmethacrylate and 50 % by weight methacrylic acid. It is also provided as a 12.5% solution (Eudragit® L 12.5). Eudragit® L 100-55 is a copolymer of 50 % by weight ethylacrylate and 50 % by weight methacrylic acid. It is also provided as 30 % dispersion (Eudragit® L 30 D-55).

(Meth)acrylic acid (co)polymers with an anionic functional group may also comprise 20 to 40 % by weight methacrylic acid and 80 to 60 % by weight methylmethacrylate. These types of polymers are usually available under the trade name Eudragit® S. It is also provided as a 12.5 % solution (Eudragit® S 12.5). Another type of methacrylic acid copolymers with an anionic functional group is available under the trade name Eudragit® FS which typically comprises 10 to 30 % by weight methylmethacrylate, 50 to 70 % by weight methylacrylate and 5 to 15 % by weight methacrylic acid. Thus, Eudragit®FS may be a polymer of 25 % by weight methylmethacrylate, 65 % by weight methylacrylate and 10 % by weight methacrylic acid. It is usually provided as 30 % dispersion (Eudragit® FS 30 D).

(Meth)acrylic acid (co)polymers with functional cationic groups may be methacrylic acid copolymers with tertiary amino groups. Such polymers may comprise 30 % to 80 % by weight of radically polymerised C₁-C₄ alkyl esters of acrylic acid or methacrylic acid and 70 to 20 % by weight methacrylate monomers with a tertiary amino group in the alkyl rest.

Suitable monomers with a functional tertiary amino group are disclosed e.g. in US 4,705,695, column 3, line 64 to column 4, line 13. They include for example dimethylaminoethyl acrylate, 2-dimethylaminopropyl acrylate, dimethylaminopropyl methacrylate, dimethylaminobenzyl acrylate, dimethylaminobenzyl methacrylate, (3-dimethylamino-2,2-dimethyl)propyl acrylate, dimethylamino-2,2-dimethylpropylmethacrylate, (3-diethylamino-2,2-dimethyl)propyl acrylate and diethylamino-2,2-dimethylpropylmethacrylate. Particularly suitable is dimethylaminoethyl methacrylate. The amount of monomers with a tertiary amino group in the copolymer may vary between 20 to 70 %, between 40 to 60 %. The amount of C₁ to C₄ alkyl esters of acrylic or methacrylic acid may be within 70 to 30 % by weight. C₁ to C₄ alcohol esters of acrylic or methacrylic acid include methylmethacrylate, ethylmethacrylate, butylmethacrylate, methylacrylate, ethylacrylate and butylacrylate. A common (meth)acrylic acid (co)polymer with a tertiary amino group may comprise 20 to 30 % by weight methylmethacrylate, 20 to 30 % by weight butylmethacrylate and 60 to 40 % by weight dimethylaminoethyl methacrylate. For example the commercially available Eudragit® E 100 comprises 25 % by weight methylmethacrylate, 25 % by weight butylmethacrylate and 50 % by weight dimethylaminoethyl methacrylate. Another common commercially available polymer, Eudragit®E PO comprises copolymers of methylmethacrylate, butylmethacrylate and dimethylaminoethyl methacrylate in a ratio of 25:25:50.

Another type of (meth)acrylic acid (co)polymers with functional cationic groups is (meth)acrylic acid (co)polymers with a quaternary amino group. This type of (meth)acrylic acid (co)polymers typically comprises 50 to 70 % of radically polymerised methylmethacrylate, 20 to 40 % by weight of ethylacrylate and 12 to 2 % by weight of 2-trimethylammoniumethyl methacrylate chloride. Such polymers are e.g. available under the trade names Eudragit®RS or Eudragit®RL.

For example, Eudragit®RS comprises radically polymerised units of 65 % by weight methylmethacrylate, 30 % by weight ethylacrylate and 5 % by weight 2-trimethylamoniumethyl methacrylate chloride. Eudragit®RL comprises radically polymerised units of 60 % by weight methylmethacrylate, 30 % by weight ethylacrylate and 10 % by weight 2-trimethylamoniumethyl methacrylate chloride.

Prolonged release matrix materials which are particularly suitable for the present invention are e.g. the neutral (meth)acrylic acid (co)polymers or the (meth)acrylic acid (co)polymers with anionic functional groups. One may for example use mixtures of these types of polymers.

For example, one may use Eudragit®NE as a neutral (meth)acrylic acid (co)polymer and Eudragit®RSPO as a (meth)acrylic acid (co)polymer with an anionic functional group. One may also use a mixture of these types of polymers.

However, one may also use a mixture of (meth)acrylic acid (co)polymers and other prolonged release matrix materials such as cellulose ethers. For example, one may use a mixture of a neutral (meth)acrylic acid (co)polymer and a hydrophobic cellulose ether. A particularly suitable example is the combination of a Eudragit®NE together with ethyl cellulose. Another prolonged release material which may be used for the present invention may be polymers such as polyethylene oxide.

As regards polyethylene oxides, particularly those polyethylene oxides with a molecular weight in the range of 1 × 10⁵ - 5 × 10⁵ may be used.

Prolonged release materials which are particularly suitable for the present invention are e.g. the neutral (meth)acrylic acid (co)polymers or the (meth)acrylic acid (co)polymers with anionic functional groups. One may for example use mixtures of these types of polymers.

For example, one may use Eudragit®NE as a neutral (meth)acrylic acid (co)polymer and Eudragit®RSPO as a (meth)acrylic acid (co)polymer with an anionic functional group. One may also use a mixture of these types of polymers.

The use of (meth)acrylic acid (co)polymers can be particularly suitable for increasing hardness/breaking strength upon heat treatment.

However, one may also use a mixture of (meth)acrylic acid (co)polymers and other prolonged release matrix materials such as cellulose ethers. For example, one may use a mixture of a neutral (meth)acrylic acid (co)polymer and a hydrophobic cellulose ether. A particularly suitable example is the combination of a Eudragit®NE together with ethyl cellulose. Another example is a mixture of cellulose ether such as hydrophobic cellulose ethers (e.g. ethyl cellulose) with a fatty alcohol (e.g. stearyl alcohol). A mixture of (meth)acrylic acid (co)polymers such as neutral (meth)acrylic acid (co)polymer (e.g. Eudragit®NE) and cellulose ethers such as hydrophobic cellulose ethers (e.g. ethyl cellulose) may also comprise a fatty alcohol (such as stearyl or cetostearyl alcohol) as a further prolonged release matrix material. Such mixtures may allow combining beneficial characteristics such as alcohol resistance and increased hardness and improved stability upon heat treatment.

The amount of prolonged release material(s) in the prolonged release formulation may be of about 5 to 90 % by weight, of about 10 to 70% by weight, of about 20 to 60 % by weight, of about 20% to about 55% by weight, of about 25% to about 50% by weight, of about 25% to about 45% by weight and preferably of about 25% to about 35% by weight based on the weight of the pharmaceutical composition. The amount of prolonged release material that is incorporated into the composition can be one way of adjusting the prolonged release properties. For example, if the amount of prolonged release material is increased, the release can be further prolonged. The aforementioned amounts refer to the overall content of prolonged release materials in a pharmaceutical composition. These amounts may thus refer to a mixture of various prolonged release materials such as a neutral (meth)acrylic acid (co)polymer, a hydrophobic cellulose ether and/or a fatty alcohol.

If cellulose ether is among the prolonged release materials, it will typically be present in an amount of about 5% to about 50% by weight, of about 5% to about 45% by weight, of about 5% to about 40% by weight, of about 5% to about 35% by weight, of about 5% to about 30% by weight, of about 5% to about 25% by weight, of about 5% to about 20% by weight such as of about 5% by weight, of about 7% by weight, of about 10% by weight, of about 15% by weight, of about 18% by weight or of about 20% by weight based on the weight of the pharmaceutical composition. An amount of 13% to 18% by weight can be preferred.

If fatty alcohol is among the prolonged release materials, it will typically be present in an amount of about 5% to about 50% by weight, of about 5% to about 45% by weight, of about 5% to about 40% by weight, of about 5% to about 35% by weight, of about 10% to about 30% by weight, of about 10% to about 25% by weight such as of about 10% by weight, of about 15% by weight, of about 20% by weight or about 25% by weight based on the weight of the pharmaceutical composition. An amount of 15% to 20% by weight can be preferred.

If (meth)acrylic acid (co)polymer is among the prolonged release materials, it will typically be present in an amount of about 5% to about 50% by weight, of about 5% to about 45% by weight, of about 5% to about 40% by weight, of about 5% to about 35% by weight, of about 10% to about 30% by weight, of about 10% to about 25% by weight such as of about 10% by weight, of about 15% by weight, of about 20% by weight or about 25% by weight based on the weight of the pharmaceutical composition.

The pharmaceutical compositions in accordance with the invention may also include pharmaceutically acceptable excipients such fillers, lubricants, binders, release rate modifiers, anti-tacking agents etc.

Fillers which may also be designated as diluents may include e.g. lactose, preferably lactose monohydrtae, glucose or saccharose, starches, their hydrolysates, microcrystalline cellulose, cellatose, sugar alcohols such as sorbitol or mannitol, polysoluble calcium salts like calcium hydrogen phosphate, dicalcium- or tricalcium phosphate and combinations of two or more of the above fillers.
Lubricants can include highly dispersed silica, talcum, corn starch, magnesium oxide and magnesium- or calcium stearate, fats like hydrated castor oil, sodium stearyl fumarate and combinations of two or more of the above lubricants.

It can be preferred to use a combination of magnesium stearate and talcum as lubricants. It has been found that if appropriate amounts of these lubricants are chosen, one can e.g. improve flow properties of granules used for compressing.

It thus can be preferred to use a lubricant amount of about 0.5% to about 4% by weight, of about 0.7% to about 3% by weight, of about 1% to about 2% by weight such as of about 1.0 % by weight, of about 1.1 % by weight, of about 1.2 % by weight, of about 1.3 % by weight, of about 1.4 % by weight, of about 1.5 % by weight, of about 1.6% by weight, of about 1.7 % by weight, of about 1.8 % by weight, of about 1.9 % by weight or of about 2.0 % by weight based on the weight of the pharmaceutical composition. An amount of about 0.75% to about 1.25% by weight based on the weight of the pharmaceutical composition can be preferred, particularly if magnesium stearate and talc are used. The aforementioned amounts refer to the amount of all lubricants (i.e. including mixtures) in the composition.

Binders can include hydroxypropyl cellulose (HPC), hydroxypropyl methyl cellulose, povidone, copovidone, polyvinyl pyrrolidone vinyl alcohol (PVPVA), microcrystalline cellulose, polyethylene glycols (PEGs), gelatine, starch or glycerol esters of fatty acids like glycerol monobehenate, glycerol palmitostearate, glycerol monostearate, and combinations thereof.

It can be preferred to use HPC as a binder as this may positively influence the stability of the tablets. The use of HPC may be particularly considered if the composition comprises 1 to 15 mg, preferably 1 to 9 mg of oxycodone HCl or an equimolar amount of a different pharmaceutically acceptable salt or of the free base and 0.5 to 7.5 mg, preferably 0.5 to 4.5 mg of naloxone HCl or an equimolar amount of a different pharmaceutically acceptable salt or of the free base. The use of oxycodone and naloxone in 2:1 ration by weight may be even more preferred in this context. In some embodiments, the composition comprises oxycodone or a pharmaceutically acceptable salt thereof in an amount equivalent to 1 mg of oxycodone HCl and naloxone or a pharmaceutically acceptable salt thereof in an amount equivalent to 0.5 mg of naloxone HCl, oxycodone or a pharmaceutically acceptable salt thereof in an amount equivalent to 2.5 mg of oxycodone HCl and naloxone or a pharmaceutically acceptable salt thereof in an amount equivalent to 1.25 mg of naloxone HCl, oxycodone or a pharmaceutically acceptable salt thereof in an amount equivalent to 5 mg of oxycodone HCl and naloxone or a pharmaceutically acceptable salt thereof in an amount equivalent to 2.5 mg of naloxone HCl, oxycodone or a pharmaceutically acceptable salt thereof in an amount equivalent to 7.5 mg of oxycodone HCl and naloxone or a pharmaceutically acceptable salt thereof in an amount equivalent to 3.75 mg of naloxone HCl. Albeit these latter dosage strengths may be preferred in some instances, HPC may also be used in higher dosage strengths. The composition thus may comprise oxycodone or a pharmaceutically acceptable salt thereof in an amount equivalent to 10 mg of oxycodone HCl and naloxone or a pharmaceutically acceptable salt thereof in an amount equivalent to 5 mg of naloxone HCl, oxycodone or a pharmaceutically acceptable salt thereof in an amount equivalent to 12.5 mg of oxycodone HCl and naloxone or a pharmaceutically acceptable salt thereof in an amount equivalent to 6.25 mg of naloxone HCl, or oxycodone or a pharmaceutically acceptable salt thereof in an amount equivalent to 15 mg of oxycodone HCl and naloxone or a pharmaceutically acceptable salt thereof in an amount equivalent to 7.5 mg of naloxone HCl. These pharmaceutical compositions may comprise at least a part of the pharmaceutically active agents in a prolonged release matrix. Such a prolonged release matrix may preferably comprise ethyl cellulose and/or a fatty alcohol as prolonged release matrix materials. However, in some embodiments the prolonged release matrix may not comprise ethyl cellulose and/or stearyl alcohol as a prolonged release matrix material. The afore-mentioned pharmaceutical composition may have less than 5% of total substances related to oxycodone and naloxone after storage under stressed conditions. Further, the afore-mentioned pharmaceutical composition may have less than 0.5% of known oxycodone or naloxone related substances after storage under stressed conditions. These pharmaceutical compositions may also provide substantially the same in vitro dissolution rate after storage under stressed conditions. Further, the pharmaceutical composition may provide the above-mentioned in vitro dissolution rates. In these pharmaceutical compositions, HPC may be used for stabilizing the formulation.

In one preferred aspect the present invention relates to the use of HPC for stabilizing a pharmaceutical composition. Such pharmaceutical compositions comprise at least part of oxycodone or a pharmaceutically acceptable salt thereof such oxycodone HCl and of naloxone or a pharmaceutically acceptable salt thereof such as naloxone HCl in a prolonged release matrix. Such pharmaceutical composition comprises oxycodone or a pharmaceutically acceptable salt thereof in an amount equivalent to 1 to 15 mg, preferably 1 to 9 mg of oxycodone HCl and naloxone or a pharmaceutically acceptable salt thereof in an amount equivalent to 0.5 to 7.5 mg, preferably 0.5 to 4.5 mg of naloxone HCl. These compositions may preferably comprise the active ingredients in 2:1 ratio by weight. Further, these compositions preferably comprise a cellulose ether such as ethyl cellulose and/or a fatty alcohol as prolonged release matrix materials. These pharmaceutical compositions may have less than 5% of total substances related to oxycodone and naloxone after storage under stressed conditions. Further, these pharmaceutical compositions may have less than 0.5% of known oxycodone or naloxone related substances after storage under stressed conditions. These pharmaceutical compositions may also provide substantially the same in vitro dissolution rate after storage under stressed conditions.

Further, the pharmaceutical composition may provide the above-mentioned in vitro dissolution rates.

It can be preferred to use povidone as a binder as this may positively influence the stability of the tablets. The use of povidone may be particularly considered if the composition comprises 10 to 160 mg, preferably 16 to 160 mg of oxycodone HCl or an equimolar amount of a different pharmaceutically acceptable salt or of the free base and 5 to 80 mg, preferably 8 to 80 mg of naloxone HCl or an equimolar amount of a different pharmaceutically acceptable salt or of the free base. The use of oxycodone and naloxone in 2:1 ratio by weight may be even more preferred in this context. In other embodiments the pharmaceutical composition comprises oxycodone or a pharmaceutically acceptable salt thereof in an amount equivalent to 10 mg of oxycodone HCl and naloxone or a pharmaceutically acceptable salt thereof in an amount equivalent to 5 mg of naloxone HCl, oxycodone or a pharmaceutically acceptable salt thereof in an amount equivalent to 20 mg of oxycodone HCl and naloxone or a pharmaceutically acceptable salt thereof in an amount equivalent to 10 mg of naloxone HCl, oxycodone or a pharmaceutically acceptable salt thereof in an amount equivalent to 30 mg of oxycodone HCl and naloxone or a pharmaceutically acceptable salt thereof in an amount equivalent to 15 mg of naloxone HCl, oxycodone or a pharmaceutically acceptable salt thereof in an amount equivalent to 40 mg of oxycodone HCl and naloxone or a pharmaceutically acceptable salt thereof in an amount equivalent to 20 mg of naloxone HCl, oxycodone or a pharmaceutically acceptable salt thereof in an amount equivalent to 60 mg of oxycodone HCl and naloxone or a pharmaceutically acceptable salt thereof in an amount equivalent to 30 mg of naloxone HCl oxycodone or a pharmaceutically acceptable salt thereof in an amount equivalent to 80 mg of oxycodone HCl and naloxone or a pharmaceutically acceptable salt thereof in an amount equivalent to 40 mg of naloxone HCl and oxycodone or a pharmaceutically acceptable salt thereof in an amount equivalent to 160 mg of oxycodone HCl and naloxone or a pharmaceutically acceptable salt thereof in an amount equivalent to 80 mg of naloxone HCl. The prolonged release matrix may in some embodiments not comprise ethyl cellulose and/or stearyl alcohol as a prolonged release matrix material.

It thus can be preferred to use a binder amount of about 1% to about 10% by weight, of about 2% to about 9% by weight, of about 3% to about 7% by weight, of about 3% to about 6% by weight, of about 3% to about 5% by weight or of about 3% to about 4% by weight such as of about 3.0 % by weight, of about 3.1 % by weight, of about 3.2 % by weight, of about 3.3 % by weight, of about 3.4 % by weight, of about 3.5 % by weight, of about 3.6% by weight, of about 3.7 % by weight, of about 3.8 % by weight, of about 3.9 % by weight or of about 3.0 % by weight based on the weight of the pharmaceutical composition. An amount of about 3.0% to about 4.0% by weight based on the weight of the pharmaceutical composition can be preferred, particularly if HPC is used as binder. The aforementioned amounts refer to the amount of all binders (i.e. including mixtures) in the composition.

It is to be understood that the functions of pharmaceutically acceptable excipients may be overlapping. For example, a spheronising agent such as microcrystalline cellulose can also be used as filler if appropriate amounts are chosen. Further, HPMC may not only act as release rate modifying agent but also as binder if e.g. used in prolonged release formulation with a coating.

Prolonged release coatings may be made from materials as they are common in the art.

They may thus be selected from e.g. hydrophobic prolonged release materials selected from (i) an alkylcellulose; (ii) an acrylic polymer; or (iii) mixtures thereof. The coating may be applied in the form of an organic or aqueous solution or dispersion.

In some embodiments, the controlled release coating is derived from an aqueous dispersion of the hydrophobic controlled release material. The coated composition can then be cured.

In preferred embodiments, the controlled release coatings include a plasticizer such as those described herein below.

In certain embodiments, one may coat with an amount of coating material which is sufficient to obtain a weight gain level from about 2 to about 20%, e.g., about 2 to about 15% and preferably about 5 to about 10% such as 6%, 7%, 8% or 9% in order to obtain sufficiently prolong the release from the formulation.

Cellulosic materials and polymers, including alkyl celluloses are prolonged release materials well suited for coating substrates, e.g., beads, granules, tablets, etc. according to the invention. Simply by way of example, one preferred alkyl cellulosic polymer is ethyl cellulose

One commercially available aqueous dispersion of ethyl cellulose is Aquacoat® such as Aquacoat® ECD30 (FMC Corp., Philadelphia, Pennsylvania, U.S.A.). Aquacoat is prepared by dissolving the ethyl cellulose in a water-immiscible organic solvent and then emulsifying the same in water in the presence of a surfactant and a stabilizer. After homogenization to generate submicron droplets, the organic solvent is evaporated under vacuum to form a pseudo latex.

Another aqueous dispersion of ethyl cellulose is commercially available as Surelease® (Colorcon, Inc., West Point, Pennsylvania, U.S.A.). This product is prepared by incorporating plasticizer into the dispersion during the manufacturing process. A hot melt of a polymer, plasticizer (dibutyl sebacate), and stabilizer (oleic acid) is prepared as a homogeneous mixture, which is then diluted with an alkaline solution to obtain an aqueous dispersion which can be applied directly onto substrates.

In other of the present invention, the prolonged release coating material is a pharmaceutically acceptable acrylic polymer, including but not limited to acrylic acid and methacrylic acid copolymers, methyl methacrylate copolymers, ethoxyethyl methacrylates, cynaoethyl methacrylate, poly(acrylic acid), poly(methacrylic acid), methacrylic acid alkylamide copolymer, poly(methyl methacrylate), polymethacrylate, poly(methyl methacrylate) copolymer, polyacrylamide, aminoalkyl methacrylate copolymer, poly(methacrylic acid anhydride) and glycidyl methacrylate copolymers.

In certain preferred embodiments, the acrylic polymer is comprised of one or more ammonium methacrylate copolymers. Ammonium methacrylate copolymers are well known in the art, and are described as fully polymerized copolymers of acrylic and methacrylic acid esters with a low content of quaternary ammonium groups. Typical examples include Eudragit® RS30D which is a low permeability ammonium methacrylate polymer and Eudragit®RL30D which is a high permeability ammonium methacrylate polymer. Eudragit RL and Eudragit RS are water swellable, and the amount of water absorbed by these polymers is pH-dependent, however, dosage forms coated with Eudragit RL and RS are pH-independent.

The acrylic coatings may comprise a mixture of two acrylic resin lacquers commercially available from Rohm Pharma under the Trade names Eudragit®RL30D and Eudragit®RS30D, respectively. The Eudragit®RL/RS dispersions of the present invention may be mixed together in any desired ration in order to ultimately obtain a prolonged-release formulation having a desirable dissolution profile.

Other polymers which can be used as a prolonged release coating materials if they are applied at sufficient amounts are e.g. hydrophilic polymers such as hyrdoxypropylmethylcellulose.

The above mentioned coatings may also be applied in combination. Further it is possible to influence the release properties of a dosage form by increasing the amount of the coating material and thus the thickness of the coating.

In embodiments of the present invention where the coating comprises an aqueous dispersion of a hydrophobic controlled release material, the inclusion of an effective amount of a plasticizer in the aqueous dispersion of hydrophobic material may further improve the physical properties of the prolonged release coating. For example, because ethyl cellulose has a relatively high glass transition temperature and may not form flexible films under normal coating conditions, it can be preferred to incorporate a plasticizer into an ethyl cellulose coating containing prolonged release coating before using the same as a coating material. Generally, the amount of plasticizer included in a coating solution is based on the concentration of the film-former, e.g., most often from about 1 to about 50 % by weight of the film-former.

Examples of suitable plasticizers for ethyl cellulose include water insoluble plasticizers such as dibutyl sebacate, diethyl phthalate, triethyl citrate, tributyl citrate, and triacetin, although it is possible that other water-insoluble plasticizers (such as acetylated monoglycerides, phthalate esters, castor oil, etc.) may be used. Triethyl citrate is an especially preferred plasticizer for the aqueous dispersions of ethyl cellulose of the present invention.

Examples of suitable plasticizers for the acrylic polymers of the present invention include, but are not limited to citric acid esters such as triethyl citrate NF XVI, tributyl citrate, dibutyl phthalate, and possibly 1,2-propylene glycol. Other plasticizers which have proved to be suitable for enhancing the elasticity of the films formed from acrylic films such as Eudragit®RL/RS lacquer solutions include polyethylene glycols, propylene glycol, diethyl phthalate, castor oil, and triacetin.

A particularly preferred aspect of the present invention relates to pharmaceutical compositions comprising at least part of oxycodone or a pharmaceutically acceptable salt thereof such oxycodone HCl and of naloxone or a pharmaceutically acceptable salt thereof such as naloxone HCl in a prolonged release matrix. The pharmaceutical composition comprises oxycodone or a pharmaceutically acceptable salt thereof in an amount equivalent to 1 to 15 mg, preferably 1 to 9 mg of oxycodone HCl and naloxone or a pharmaceutically acceptable salt thereof in an amount equivalent to 0.5 to 7.5 mg, preferavly 0.5 to 4.5 mg of naloxone HCl. Further, the pharmaceutical composition comprises HPC as a binder and/or a stabilizing agent.

These compositions may preferably comprise the active ingredients in 2:1 ratio by weight. Further, these compositions preferably comprise a cellulose ether such as ethyl cellulose and/or a fatty alcohol as prolonged release matrix materials. These pharmaceutical compositions may have less than 5% of total substances related to oxycodone and naloxone after storage under stressed conditions. Further, these pharmaceutical compositions may have less than 0.5% of known oxycodone or naloxone related substances after storage under stressed conditions. These pharmaceutical compositions may also provide substantially the same in vitro dissolution rate after storage under stressed conditions. Further, the pharmaceutical composition may provide the above-mentioned in vitro dissolution rates.

The pharmaceutical compositions in accordance with the invention may be manufactured by mixing the pharmaceutically active agents with the pharmaceutically acceptable excipients and in particular the polonged release material(s), the binder, the filler etc.

This mixture can then be melt-extruded to obtain monolithic compositions or multiparticulates. The multiparticulates may be used directly as multiparticulates, filled into capsules or further compressed into tablets.

Alternatively the mixture may be granulated e.g. by fluidized bed granulation and the granules may be used directly as granules, filled into capsules or further compressed into tablets.

The present invention also relates to such methods of manufacturing.

The present invention also relates to the use of HPC for manufacturing and/or stabilizing the above pharmaceutical compositions.

The invention is now illustrated with respect to specific examples. These examples are, however, not to be construed as limiting.

### EXAMPLES

### Example 1:

Tablets of the composition as shown in Table 1 were manufactured.

| Tablet | OXN 219/06 | OXN 10/5 |
|---|---|---|
| Ingredient | Amount (mg) | Amount (mg) |
| Oxycodone HCI | 5.00 | 10.00 |
| Naloxone HCI | 2.50 | 5.00 |
| Povidone K30 | 5.00 | 5.00 |
| Lactose monohydrate | 71.75 | 64.25 |
| Ethyl cellulose | 10.00 | 10.00 |
| Stearyl alcohol | 25.00 | 25.00 |
| Talc | 2.50 | 2.50 |
| Mg-Stearate | 1.25 | 1.25 |
| | | |
| Core weight | 123.0 | 114.0 |
| Water content | 0.5 | |
| | | |

| | | |
|---|---|---|
| The amounts indicated refer to the amounts of solids used. | | |

Oxycodone hydrochloride, naloxone hydrochloride, povidone K30, ethyl cellulose N45, stearyl alcohol and lactose monohydrate were blended in a tumbler mixer.

Subsequently, the blend was extruded in a Leistritz Micro 18 or Leistritz Micro 27 twin-screw extruder without paddle means and with a die plate. The temperature profiles are provided in the following table.

**Table 2 Temperature profile for a Leistritz Micro 18 and Micro 27**

| Extrudertyp: | Micro 18 | Micro 27 |
|---|---|---|
| Heating zone 1 (feeding zone) | 25°C | 25°C |
| Heating zone 2 | 55°C | 55°C |
| Heating zone 3 | 63°C | 83°C |
| Heating zone 4 | 63°C | 83°C |
| Heating zone 5 | 60°C | 80°C |
| Heating zone 6 | 60°C | 80°C |
| Heating zone 7 | 60°C | 80°C |
| Heating zone 8 | 60°C | 80°C |
| Heating zone 9 | n.a. | 80°C |
| Heating zone 10 | n.a. | 80°C |
| Die head | 60°C | 60°C |

Subsequently the extrudate was cooled and milled to obtain granules.
The granules were then blended in a tumbler mixer with magnesium stearate and talc and compressed into tablets using a rotary press.

Tablets OXN 219/06 were then analysed as regards in vitro release behavior using the Ph. European paddle method at 50 rpm in 500 ml simulated gastric fluid (SGFₛₚ) dissolution medium (0.1 N HCl with pH 1.2 and 2 g sodium chloride per litre). Aliquots of the dissolution media are withdrawn at the respective time points and analysed by HPLC at 230 nm.

The in vitro release data is indicated as percentage (based on the label content of active tested) in table 3. The upper and lower limits of the desired *in vitro* release range are indicated.

**Table 3**

| | | | Tablets | PN3450 | |
|---|---|---|---|---|---|
| | | | Dissolution medium | SGFₛₚ | |
| time (min) | min. limit | max. limit | | Oxy | Nal |
| 15 | 10% | 30% | | 18% | 18% |
| 120 | 40% | 60% | | 50% | 50% |
| 600 | 80% | | | 94% | 94% |

| | | | | | |
|---|---|---|---|---|---|
| Oxy = oxycodone HCl, Nal = naloxone HCl. min. limit: indicates the lower limit of the amount of oxycodone that should be release at this point in time, max. limit: indicates the upper limit of the amount of oxycodone that should be release at this point in time | | | | | |

This formulation showed good results regarding the desired dissolution profile. Subsequently, the tablets were stored for 1 month at 60°C and 80% relative humidity (rH) and the amount of total related substances was measured. This was done by grinding the tablets and extracting oxyocodone HCl and naloxone HCl. To this end, the powder was put in a vessel and 1 ml of 1% Na₂EDTA and 10 ml of methanol were added. Subsequently, the sample was treated by ultrasonication for 30min. Then, 0.002M H₂SO₄ was added up to 100 ml. The solution was filtrated and analyzed by HPLC using Hypersil BDS C18, 3µ, 3.0*100 mm from Thermo Finigan at 210 nm.

Total related substances for this batch: 5.98%
Noroxymorphone (N1): 1.14%.

In a next step, the substances responsible for the impurities were identified. To this end, tablets of comparable composition were manufactured except for the presence of one of the excipients. These tablets were stored for 1 month at 60°C and 80% relative humidity (rH) and the amount of total related substances was measured as described above. The results are depicted in Figure 1. These results suggested that the impurities resulted from the binder povidone.

### Example 2:

Tablets of the composition as shown in Table 4 were manufactured.

**Table 4**

| Tablet | OXN 226/06 | OXN 231/06 |
|---|---|---|
| Ingredient | Amount (mg) | Amount (mg) |
| Oxycodone HCI | 5.00 mg | 5.00 mg |
| Naloxone HCI | 2.50 mg | 2.50 mg |
| Hydroxypropyl cellulose | 5.00 mg | 5.00 mg |
| Lactose monohydrate | 71.75 mg | 71.75 mg |
| Ethyl cellulose | 20.00 mg | 20.00 mg |
| Stearyl alcohol | 25.00 mg | 25.00 mg |
| Talc | 2.50 mg | 2.50 mg |
| Mg-Stearate | 1.25 mg | 1.25 mg |
| | | |
| Core weight | 133.0 mg | 133.0 mg |
| Water content | 0.5 mg | 0.5 mg |

| | | |
|---|---|---|
| The amounts indicated refer to the amounts of solids used. | | |

The tablets were manufactured as described in example 1.

Tablets OXN 226/06 and 231/06 were then analysed as regards in vitro release behavior using the Ph. European paddle method at 50 rpm in 500 ml simulated gastric fluid (SGFₛₚ) dissolution medium . Aliquots of the dissolution media are withdrawn at the respective time points and analysed by HPLC at 230 nm.

The in vitro release data is indicated as percentage (based on the label content of active tested) in table 5. The upper and lower limits of the desired *in vitro* release range are indicated.

**Table 5**

| | | | Tablets | OXN 226/06 | | OXN231/06 | |
|---|---|---|---|---|---|---|---|
| | | | Dissolution medium | SGFₛₚ | | SGFₛₚ | |
| time (min) | min. limit | max. limit | | Oxy | Nal | Oxy | Nal |
| 15 | 10% | 30% | | 21% | 21% | 22% | 23% |
| 120 | 40% | 60% | | 56% | 56% | 59% | 60% |
| 600 | 80% | | | 96% | 96% | 95% | 94% |

| | | | | | | | |
|---|---|---|---|---|---|---|---|
| Oxy = oxycodone HCl, Nal = naloxone HCl. min. limit: indicates the lower limit of the amount of oxycodone that should be release at this point in time, max. limit: indicates the upper limit of the amount of oxycodone that should be release at this point in tim | | | | | | | |

Tablets 231/06 were then film coated with Opadry blue 85F30569 as described in table 5 below:

**Table 6**

| Main Coating parameters | OXN/2.5-231A-06 |
|---|---|
| Amount of filmcoat (Target) | 3 % of core weight |
| Inlet air volume | 360 m³/h |
| rpm of Coater pan | 25 |
| Sprayrate | 15 g/min. |
| Spray pressure | 1.2 bar |
| Outlet air temp. | 33°C |

Film coated tablets OXN 231/06 were then analysed as regards in vitro release behavior using the Ph. European paddle method at 50 rpm in 500 ml simulated gastric fluid (SGFₛₚ) dissolution medium. Aliquots of the dissolution media are withdrawn at the respective time points and analysed by HPLC at 230 nm.

The in vitro release data is indicated as percentage (based on the label content of active tested) in table 7. The upper and lower limits of the desired *in vitro* release range are indicated.

**Table 7**

| | | | Tablets | OXN231/06 | |
|---|---|---|---|---|---|
| | | | Dissolution medium | SGFₛₚ | |
| time (min) | min. limit | max. limit | | Oxy | Nal |
| 15 | 10% | 30% | | 19% | 19% |
| 120 | 40% | 60% | | 53% | 53% |
| 600 | 80% | | | 94% | 96% |

| | | | | | |
|---|---|---|---|---|---|
| Oxy = oxycodone HCl, Nal = naloxone HCl. min. limit: indicates the lower limit of the amount of oxycodone that should be release at this point in time, max. limit: indicates the upper limit of the amount of oxycodone that should be release at this point in time | | | | | |

Subsequently, the tablets were stored for 1 month at 40°C and 75% relative humidity (rH) and the amount of total related substances was measured as described in example 1.

Total related substance for this batch: 0.5%

### Example 3:

Tablets of the composition as shown in Table 8 were manufactured.

**Table 8**

| Tablet | OXN 250/07 | OXN 261/07 |
|---|---|---|
| Ingredient | Amount (mg) | Amount (mg) |
| Oxycodone HCI | 5.00 | 5.00 |
| Naloxone HCI | 2.50 | 2.50 |
| Hydroxypropyl cellulose | 5.00 | 5.00 |
| Lactose monohydrate | 71.75 | 71.75 |
| Ethyl cellulose | 20.00 | 20.00 |
| Stearyl alcohol | 25.00 | 25.00 |
| Talc | 2.50 | 2.50 |
| Mg-Stearate | 1.25 | 1.25 |
| Water content of API | 0.50 | 0.50 |
| | | |
| Core weight | 133.50 | 133.5 |
| | | |
| Opadry blue 85F30569 | 4.00 | 4.00 |
| Talc | 0.10 | 0.10 |
| | | |
| Final tablet weight | 137.6 | 137.6 |

| | | |
|---|---|---|
| The amounts indicated refer to the amounts of solids used. API = active pharmaceutical ingredient | | |

The tablets were manufactured as described in example 1.

The tablets were then coated with Opadry blue 85F30569. The coating parameters were as in table 9.

**Table 9**

| Main Coating parameters | |
|---|---|
| | |
| Amount of film coat (Target) | 3 % of core weight |
| Inlet air volume | 360 m³/h |
| rpm of Coater pan | 15 |
| Initial Sprayrate | 15 ml/min. |
| Main Sprayrate | 20 ml/min. |
| Spray pressure | 1.5 bar |
| Outlet air temp. | 38°C |

Tablets OXN 250/07 and 261/07 were then analysed as regards in vitro release behavior using the Ph. European paddle method at 50 rpm in 500 ml simulated gastric fluid (SGFₛₚ) dissolution medium. Aliquots of the dissolution media are withdrawn at the respective time points and analysed by HPLC at 230 nm.

The in vitro release data is indicated as percentage (based on the label content of active tested) in table 10. The upper and lower limits of the desired *in vitro* release range are indicated.

**Table 10**

| | | | Tablets | OXN 250/07 | | OXN261/07 | |
|---|---|---|---|---|---|---|---|
| | | | Dissolution medium | SGFₛₚ | | SGFₛₚ | |
| time (min) | min. limit | max. limit | | Oxy | Nal | Oxy | Nal |
| 15 | 10% | 30% | | 18% | 18% | 19% | 19% |
| 120 | 40% | 60% | | 52% | 51% | 54% | 54% |
| 600 | 80% | | | 97% | 96% | 99% | 97% |

| | | | | | | | |
|---|---|---|---|---|---|---|---|
| Oxy = oxycodone HCl, Nal = naloxone HCl. min. limit: indicates the lower limit of the amount of oxycodone that should be release at this point in time, max. limit: indicates the upper limit of the amount of oxycodone that should be release at this point in time | | | | | | | |

Tablets OXN 250/07 were then stored for 3 months at 25°C and 60% rH, for 3 months at 30°C and 65% rH or for 3 months at 40°C and 60% rH. Subsequently, OXN 250/07 were analysed as regards in vitro release behavior using the Ph. European paddle method at 50 rpm in 500 ml simulated gastric fluid (SGFₛₚ) dissolution medium. Aliquots of the dissolution media are withdrawn at the respective time points and analysed by HPLC at 230 nm.

The in vitro release data is indicated as percentage (based on the label content of active tested) in tables 11 and 12. The upper and lower limits of the desired *in vitro* release range are indicated.

**Table 11**

| | | | Tablets | OXN 250/07 | | OXN 250/07 | |
|---|---|---|---|---|---|---|---|
| | | | Dissolution medium | SGFₛₚ | | SGFₛₚ | |
| | | | Storage condition | initial | | 3 months@35°C, 60%rH | |
| time (min) | min. limit | max. limit | | Oxy | Nal | Oxy | Nal |
| 15 | 10% | 30% | | 18% | 17% | 19% | 14% |
| 120 | 40% | 60% | | 52% | 50% | 52% | 48% |
| 600 | 80% | | | 97% | 94% | 97% | 94% |

| | | | | | | | |
|---|---|---|---|---|---|---|---|
| Oxy = oxycodone HCl, Nal = naloxone HCl. min. limit: indicates the lower limit of the amount of oxycodone that should be release at this point in time, max. limit: indicates the upper limit of the amount of oxycodone that should be release at this point in time | | | | | | | |

**Table 12**

| | | | Tablets | OXN 250/07 | | OXN 250/07 | |
|---|---|---|---|---|---|---|---|
| | | | Dissolution medium | SGFₛₚ | | SGFₛₚ | |
| | | | Storage condition | 3 months@30°C, 65%rH | | 3 months@40°C, 60%rH | |
| time (min) | min. limit | max. limit | | Oxy | Nal | Oxy | Nal |
| 15 | 10% | 30% | | 17% | 16% | 16% | 12% |
| 120 | 40% | 60% | | 50% | 49% | 49% | 44% |
| 600 | 80% | | | 96% | 93% | 93% | 88% |

| | | | | | | | |
|---|---|---|---|---|---|---|---|
| Oxy = oxycodone HCl, Nal = naloxone HCl. min. limit: indicates the lower limit of the amount of oxycodone that should be release at this point in time, max. limit: indicates the upper limit of the amount of oxycodone that should be release at this point in time | | | | | | | |

The amount of total related substances was measured as described in example 1. The results are shown in Table 13.

**Table 13**

| Tablet | OXN250/07 | OXN250/07 | OXN250/07 | OXN250/07 |
|---|---|---|---|---|
| Storage conditions | Initial | 3months @25°C/60%/rH | 3months @30°C/65%rH | 3months @ 40°C/60%rH |
| Noroxymor phone | 0.06% | 0.13% | 0.12% | 0.16% |
| Total Impurities | 0.35% | 0.77% | 0.56% | 0.73% |

### Example 8

Tablets of the composition as shown in Table 15 were manufactured.

**Table 14**

| Tablet | OXN10/5 | OXN20/10 | OXN40/20 |
|---|---|---|---|
| Ingredient | Amount (mg) | Amount (mg) | Amount (mg) |
| Oxycodone HCl | 10.5 mg | 21.00 mg | 42.00 mg |
| Naloxone HCl | 5.45 mg | 10.9 mg | 21.8 mg |
| Povidone K30 | 5.0 mg | 7.25 mg | 14.5 mg |
| Ethyl cellulose N45 | 10.0 mg | 12.0 mg | 24.0 mg |
| Stearyl alcohol | 25.0 mg | 29.5 mg | 59.0 mg |
| Lactose monohydrate | 64.25 mg | 54.5 mg | 109.0 mg |
| Talc | 2.5 mg | 2.5 mg | 5.0 mg |
| Mg stearate | 1.25 mg | 1.25 mg | 2.5 mg |
| Total core | 123 mg | 138 mg | 277 mg |

| | | | |
|---|---|---|---|
| The amounts indicated refer to the amounts of solids used.; | | | |

First, oxycodone HCl, naloxone HCl, povidone K30,ethyl cellulose N45, stearyl alcohol and lactose monohydrate were blended in a tumbling mixer. Then the blend was extruded using a Leistritz Micro 18 or Leistritz Micro 27 twin-screw extruder (not a planetary roller extruder) without paddle means and with a die plate.

The temperature profile is provided in the following table 14.

**Table 15**

| Extrudertyp: | Micro 18 | Micro 27 |
|---|---|---|
| Heating zone 1 (feeding zone) | 25°C | 25°C |
| Heating zone 2 | 55°C | 55°C |
| Heating zone 3 | 63°C | 83°C |
| Heating zone 4 | 63°C | 83°C |
| Heating zone 5 | 60°C | 80°C |
| Heating zone 6 | 60°C | 80°C |
| Heating zone 7 | 60°C | 80°C |
| Heating zone 8 | 60°C | 80°C |
| Heating zone 9 | n.a. | 80°C |
| Heating zone 10 | n.a. | 80°C |
| Die head | 60°C | 60°C |

The extrudate was cooled, milled and blended with Mg stearate und talc in a tumbler mixer. Subsequently, the granules compressed in a rotary press to form tablets.

### Example 9:

Tablets of the composition as shown in Table 16 were manufactured.

**Table 16**

| Tablet | OXN 315/10 |
|---|---|
| Ingredient | Amount (mg) |
| Oxycodone HCI | 2.50 |
| Naloxone HCI | 1.25 |
| Hydroxypropyl cellulose | 2.50 |
| Lactose monohydrate | 35.88 |
| Ethyl cellulose | 10.00 |
| Stearyl alcohol | 12.50 |
| Talc | 1.25 |
| Mg-Stearate | 0.63 |
| Water content of API | 0.24 |
| | |
| Core weight | 66.75 |
| | |
| Opadry II yellow 85F2200026 | 2.00 |
| Talc | 0.07 |
| | |
| Final tablet weight | 68.82 |

| | |
|---|---|
| The amounts indicated refer to the amounts of solids used. API = active pharmaceutical ingredient | |

The tablets were manufactured as described in example 1 and coated as described in example 3.

Tablets OXN 315/10 were then analysed as regards in vitro release behavior using the Ph. European paddle method at 50 rpm in 500 ml simulated gastric fluid (SGFₛₚ) dissolution medium. Aliquots of the dissolution media are withdrawn at the respective time points and analysed by HPLC at 230 nm.

The in vitro release data is indicated as percentage (based on the label content of active tested) in table 17. The upper and lower limits of the desired *in vitro* release range are indicated.

**Table 17**

| | | | Tablets | OXN 315/10 | |
|---|---|---|---|---|---|
| | | | Dissolution medium | SGFₛₚ | |
| time (min) | min. limit | max. limit | | Oxy | Nal |
| 15 | 10% | 30% | | 22% | 22% |
| 120 | 40% | 60% | | 61% | 60% |
| 600 | 80% | | | 101% | 101% |

| | | | | | |
|---|---|---|---|---|---|
| Oxy = oxycodone HCl, Nal = naloxone HCl. min. limit: indicates the lower limit of the amount of oxycodone that should be released at this point in time, max. limit: indicates the upper limit of the amount of oxycodone that should be released at this point in time | | | | | |

Tablets OXN 315/10 were then stored for 3, 6, and 9 months at 25°C and 60% rH, for 3, 6 and 9 months at 30°C and 75% rH or for 3 and 6 months at 40°C and 75% rH. Subsequently, OXN 315/10 were analysed as regards in vitro release behavior using the Ph. European paddle method at 50 rpm in 500 ml simulated gastric fluid (SGFₛₚ) dissolution medium. Aliquots of the dissolution media are withdrawn at the respective time points and analysed by HPLC at 230 nm.

The in vitro release data is indicated as percentage (based on the label content of active tested) in tables 18, 19 and 20 for 6 months of storage to allow for a comparison. The upper and lower limits of the desired *in vitro* release range are indicated.

**Table 18**

| | | | Tablets | OXN 315/10 | | OXN 315/10 | |
|---|---|---|---|---|---|---|---|
| | | | Dissolution medium | SGFₛₚ | | SGFₛₚ | |
| | | | Storage condition | Initial | | 6 months@25°C, 60%rH | |
| time (min) | min. limit | max. limit | | Oxy | Nal | Oxy | Nal |
| 15 | 10% | 30% | | 22% | 22% | 20% | 20% |
| 120 | 40% | 60% | | 61% | 60% | 60% | 59% |
| 600 | 80% | | | 101% | 101% | 101% | 101% |

| | | | | | | | |
|---|---|---|---|---|---|---|---|
| Oxy = oxycodone HCl, Nal = naloxone HCl. min. limit: indicates the lower limit of the amount of oxycodone that should be released at this point in time, max. limit: indicates the upper limit of the amount of oxycodone that should be released at this point in time | | | | | | | |

**Table 19**

| | | | Tablets | OXN 315/10 | | OXN 315/10 | |
|---|---|---|---|---|---|---|---|
| | | | Dissolution medium | SGFₛₚ | | SGFₛₚ | |
| | | | Storage condition | Initial | | 6 months@30°C, 75%rH | |
| time (min) | min. limit | max. limit | | Oxy | Nal | Oxy | Nal |
| 15 | 10% | 30% | | 22% | 22% | 20% | 20% |
| 120 | 40% | 60% | | 61% | 60% | 58% | 58% |
| 600 | 80% | | | 101% | 101% | 102% | 102% |

| | | | | | | | |
|---|---|---|---|---|---|---|---|
| Oxy = oxycodone HCl, Nal = naloxone HCl. min. limit: indicates the lower limit of the amount of oxycodone that should be released at this point in time, max. limit: indicates the upper limit of the amount of oxycodone that should be released at this point in time | | | | | | | |

**Table 20**

| | | | Tablets | OXN 315/10 | | OXN 315/10 | |
|---|---|---|---|---|---|---|---|
| | | | Dissolution medium | SGFₛₚ | | SGFₛₚ | |
| | | | Storage condition | Initial | | 6 months@40°C, 75%rH | |
| time (min) | min. limit | max. limit | | Oxy | Nal | Oxy | Nal |
| 15 | 10% | 30% | | 22% | 22% | 20% | 18% |
| 120 | 40% | 60% | | 61% | 60% | 56% | 54% |
| 600 | 80% | | | 101% | 101% | 97% | 96% |

| | | | | | | | |
|---|---|---|---|---|---|---|---|
| Oxy = oxycodone HCl, Nal = naloxone HCl. min. limit: indicates the lower limit of the amount of oxycodone that should be released at this point in time, max. limit: indicates the upper limit of the amount of oxycodone that should be released at this point in time | | | | | | | |

The amount of total related substances was measured as described in example 1. The results are shown in Table 21. Again the comparison for 6 months of storage is shown.

**Table 21**

| Tablet | OXN315/10 | OXN315/10 | OXN315/10 | OXN315/10 |
|---|---|---|---|---|
| Storage conditions | Initial | 6months @25°C/60%rH | 6months @30°C/75%rH | 6months @40°C/75%rH |
| Noroxymor phone | 0.08% | 0.11% | 0.11% | 0.24% |
| Total Impurities | 0.3% | 0.5% | 0.5% | 1.2% |

The amount of noroxymorphone was the highest of all know related substances and below 0.24% under any storage conditions tested.

The amount of total impurities was below 1.2% under any storage conditions tested.

### Example 10:

Tablets of the composition as shown in Table 22 were manufactured.

**Table 22**

| Tablet | OXN 333/10 |
|---|---|
| Ingredient | Amount (mg) |
| Oxycodone HCI | 15.00 |
| Naloxone HCI | 7.50 |
| Hydroxypropyl cellulose | 5.00 |
| Lactose monohydrate | 55.80 |
| Ethyl cellulose | 20.00 |
| Stearyl alcohol | 25.00 |
| Talc | 2.50 |
| Mg-Stearate | 1.25 |
| Water content of API | 1.48 |
| | |
| Core weight | 133.48 |
| | |
| Opadry II grey 85F275005 | 4.00 |
| Talc | 0.15 |
| | |
| Final tablet weight | 137,63 |

| | |
|---|---|
| The amounts indicated refer to the amounts of solids used. API = active pharmaceutical ingredient | |

The tablets were manufactured as described in example 1 and coated as described in example 3.

Tablets OXN 333/10 were then analysed as regards in vitro release behavior using the Ph. European paddle method at 50 rpm in 500 ml simulated gastric fluid (SGFₛₚ) dissolution medium. Aliquots of the dissolution media are withdrawn at the respective time points and analysed by HPLC at 230 nm.

The in vitro release data is indicated as percentage (based on the label content of active tested) in table 23. The upper and lower limits of the desired *in vitro* release range are indicated.

**Table 23**

| | | | Tablets | OXN 333/10 | |
|---|---|---|---|---|---|
| | | | Dissolution medium | SGFₛₚ | |
| time (min) | min. limit | max. limit | | Oxy | Nal |
| 15 | 10% | 30% | | 20% | 21% |
| 120 | 45% | 65% | | 57% | 58% |
| 600 | 80% | | | 99% | 99% |

| | | | | | |
|---|---|---|---|---|---|
| Oxy = oxycodone HCl, Nal = naloxone HCl. min. limit: indicates the lower limit of the amount of oxycodone that should be released at this point in time, max. limit: indicates the upper limit of the amount of oxycodone that should be released at this point in time | | | | | |

Tablets OXN 333/10 were then stored for 3 and 6 months at 25°C and 60% rH, for 3, and 6 months at 30°C and 75% rH or for 3 and 6 months at 40°C and 75% rH. Subsequently, OXN 333/10 were analysed as regards in vitro release behavior using the Ph. European paddle method at 50 rpm in 500 ml simulated gastric fluid (SGFₛₚ) dissolution medium. Aliquots of the dissolution media are withdrawn at the respective time points and analysed by HPLC at 230 nm.

The in vitro release data is indicated as percentage (based on the label content of active tested) in tables 24, 25 and 26 for 6 months of storage to allow for a comparison. The upper and lower limits of the desired *in vitro* release range are indicated.

**Table 24**

| | | | Tablets | OXN 333/10 | | OXN 333/10 | |
|---|---|---|---|---|---|---|---|
| | | | Dissolution medium | SGFₛₚ | | SGFₛₚ | |
| | | | Storage condition | Initial | | 6 months@25°C, 60%rH | |
| time (min) | min. limit | max. limit | | Oxy | Nal | Oxy | Nal |
| 15 | 10% | 30% | | 20% | 21% | 20% | 19% |
| 120 | 45% | 65% | | 57% | 58% | 57% | 56% |
| 600 | 80% | | | 99% | 99% | 98% | 98% |

| | | | | | | | |
|---|---|---|---|---|---|---|---|
| Oxy = oxycodone HCl, Nal = naloxone HCl. min. limit: indicates the lower limit of the amount of oxycodone that should be released at this point in time, max. limit: indicates the upper limit of the amount of oxycodone that should be released at this point in time | | | | | | | |

**Table 25**

| | | | Tablets | OXN 333/10 | | OXN 333/10 | |
|---|---|---|---|---|---|---|---|
| | | | Dissolution medium | SGFₛₚ | | SGFₛₚ | |
| | | | Storage condition | Initial | | 6 months@30°C, 75%rH | |
| time (min) | min. limit | max. limit | | Oxy | Nal | Oxy | Nal |
| 15 | 10% | 30% | | 20% | 21% | 19% | 19% |
| 120 | 45% | 65% | | 57% | 58% | 56% | 55% |
| 600 | 80% | | | 99% | 99% | 99% | 98% |

| | | | | | | | |
|---|---|---|---|---|---|---|---|
| Oxy = oxycodone HCl, Nal = naloxone HCl. min. limit: indicates the lower limit of the amount of oxycodone that should be released at this point in time, max. limit: indicates the upper limit of the amount of oxycodone that should be released at this point in time | | | | | | | |

**Table 26**

| | | | Tablets | OXN 333/10 | | OXN 333/10 | |
|---|---|---|---|---|---|---|---|
| | | | Dissolution medium | SGFₛₚ | | SGFₛₚ | |
| | | | Storage condition | Initial | | 6 months@40°C, 75%rH | |
| time (min) | min. limit | max. limit | | Oxy | Nal | Oxy | Nal |
| 15 | 10% | 30% | | 20% | 21% | 18% | 18% |
| 120 | 45% | 65% | | 57% | 58% | 53% | 52% |
| 600 | 80% | | | 99% | 99% | 96% | 94% |

| | | | | | | | |
|---|---|---|---|---|---|---|---|
| Oxy = oxycodone HCl, Nal = naloxone HCl. min. limit: indicates the lower limit of the amount of oxycodone that should be released at this point in time, max. limit: indicates the upper limit of the amount of oxycodone that should be released at this point in time | | | | | | | |

The amount of total related substances was measured as described in example 1. The results are shown in Table 27. Again the comparison for 6 months of storage is shown.

**Table 27**

| Tablet | OXN333/10 | OXN333/10 | OXN333/10 | OXN333/10 |
|---|---|---|---|---|
| Storage conditions | Initial | 6months @25°C/60%orH | 6months @30°C/75%rH | 6months @40°C/75%rH |
| Noroxymor phone | 0.04% | 0.07% | 0.07% | 0.10% |
| Total Impurities | 0.1% | 0.1% | 0.1% | 0.4% |

The amount of noroxymorphone was the highest of all know related substances and below 0.10% under any storage conditions tested.

The amount of total impurities was below 0.4% under any storage conditions tested.

### Example 11:

Tablets of the composition as shown in Table 28 were manufactured.

**Table 28**

| Tablet | OXN 335/10 |
|---|---|
| Ingredient | Amount (mg) |
| Oxycodone HCI | 30.00 |
| Naloxone HCI | 15.00 |
| Polyvinylpovidone | 7.25 |
| Lactose monohydrate | 38.55 |
| Ethyl cellulose | 12.00 |
| Stearyl alcohol | 29.50 |
| Talc | 2.50 |
| Mg-Stearate | 1.25 |
| Water content of API | 2.85 |
| | |
| Core weight | 138.90 |
| | |
| Opadry II brown 85F265210 | 4.00 |
| Talc | 0.31 |
| | |
| Final tablet weight | 143.21 |

| | |
|---|---|
| The amounts indicated refer to the amounts of solids used. API = active pharmaceutical ingredient | |

The tablets were manufactured as described in example 8 and coated as described in example 3.

Tablets OXN 335/10 were then analysed as regards in vitro release behavior using the Ph. European paddle method at 50 rpm in 500 ml simulated gastric fluid (SGFₛₚ) dissolution medium. Aliquots of the dissolution media are withdrawn at the respective time points and analysed by HPLC at 230 nm.

The in vitro release data is indicated as percentage (based on the label content of active tested) in table 29. The upper and lower limits of the desired *in vitro* release range are indicated.

**Table 29**

| | | | Tablets | OXN 335/10 | |
|---|---|---|---|---|---|
| | | | Dissolution medium | SGFₛₚ | |
| time (min) | min. limit | max. limit | | Oxy | Nal |
| 15 | 10% | 30% | | 19% | 19% |
| 120 | 45% | 65% | | 55% | 56% |
| 600 | 80% | | | 97% | 97% |

| | | | | | |
|---|---|---|---|---|---|
| Oxy = oxycodone HCl, Nal = naloxone HCl. min. limit: indicates the lower limit of the amount of oxycodone that should be released at this point in time, max. limit: indicates the upper limit of the amount of oxycodone that should be released at this point in time | | | | | |

Tablets OXN 335/10 were then stored for 3 and 6 months at 25°C and 60% rH, for 3, and 6 months at 30°C and 75% rH or for 3 and 6 months at 40°C and 75% rH. Subsequently, OXN 335/10 were analysed as regards in vitro release behavior using the Ph. European paddle method at 50 rpm in 500 ml simulated gastric fluid (SGFₛₚ) dissolution medium. Aliquots of the dissolution media are withdrawn at the respective time points and analysed by HPLC at 230 nm.

The in vitro release data is indicated as percentage (based on the label content of active tested) in tables 30, 31 and 32 for 6 months of storage to allow for a comparison. The upper and lower limits of the desired *in vitro* release range are indicated.

**Table 30**

| | | | Tablets | OXN 335/10 | | OXN 335/10 | |
|---|---|---|---|---|---|---|---|
| | | | Dissolution medium | SGFₛₚ | | SGFₛₚ | |
| | | | Storage condition | Initial | | 6 months@25°C, 60%rH | |
| time (min) | min. limit | max. limit | | Oxy | Nal | Oxy | Nal |
| 15 | 10% | 30% | | 19% | 19% | 21% | 21% |
| 120 | 45% | 65% | | 55% | 56% | 57% | 58% |
| 600 | 80% | | | 97% | 97% | 98% | 98% |

| | | | | | | | |
|---|---|---|---|---|---|---|---|
| Oxy = oxycodone HCl, Nal = naloxone HCl. min. limit: indicates the lower limit of the amount of oxycodone that should be released at this point in time, max. limit: indicates the upper limit of the amount of oxycodone that should be released at this point in time | | | | | | | |

**Table 31**

| | | | Tablets | OXN 335/10 | | OXN 335/10 | |
|---|---|---|---|---|---|---|---|
| | | | Dissolution medium | SGFₛₚ | | SGFₛₚ | |
| | | | Storage condition | Initial | | 6 months@30°C, 75%rH | |
| time (min) | min. limit | max. limit | | Oxy | Nal | Oxy | Nal |
| 15 | 10% | 30% | | 19% | 19% | 21% | 20% |
| 120 | 45% | 65% | | 55% | 56% | 49% | 59% |
| 600 | 80% | | | 97% | 97% | 99% | 99% |

| | | | | | | | |
|---|---|---|---|---|---|---|---|
| Oxy = oxycodone HCl, Nal = naloxone HCl. min. limit: indicates the lower limit of the amount of oxycodone that should be released at this point in time, max. limit: indicates the upper limit of the amount of oxycodone that should be released at this point in time | | | | | | | |

**Table 32**

| | | | Tablets | OXN 335/10 | | OXN 335/10 | |
|---|---|---|---|---|---|---|---|
| | | | Dissolution medium | SGFₛₚ | | SGFₛₚ | |
| | | | Storage condition | Initial | | 6 months@40°C, 75%rH | |
| time (min) | min. limit | max. limit | | Oxy | Nal | Oxy | Nal |
| 15 | 10% | 30% | | 19% | 19% | 23% | 22% |
| 120 | 45% | 65% | | 55% | 56% | 64% | 63% |
| 600 | 80% | | | 97% | 97% | 99% | 98% |

| | | | | | | | |
|---|---|---|---|---|---|---|---|
| Oxy = oxycodone HCl, Nal = naloxone HCl. min. limit: indicates the lower limit of the amount of oxycodone that should be released at this point in time, max. limit: indicates the upper limit of the amount of oxycodone that should be released at this point in time | | | | | | | |

The amount of total related substances was measured as described in example 1. The results are shown in Table 33. Again the comparison for 6 months of storage is shown.

**Table 33**

| Tablet | OXN335/10 | OXN335/10 | OXN335/10 | OXN335/10 | |
|---|---|---|---|---|---|
| Storage conditions | Initial | 6months @25°C/60%rH | 6months @30°C/75%rH | 6months @40°C/75%rH | |
| Noroxymor phone | 0.04% | 0.10% | 0.13% | 0.22% | |
| Total Impurities | 0.1% | 0.2% | 0.5% | 1.5% | |

The amount of noroxymorphone was the highest of all know related substances and below 0.22% under any storage conditions tested.

The amount of total impurities was below 1.5% under any storage conditions tested.

### Example 12:

Tablets of the composition as shown in Table 34 were manufactured.

**Table 34**

| Tablet | OXN 336/10 |
|---|---|
| Ingredient | Amount (mg) |
| Oxycodone HCI | 60.00 |
| Naloxone HCI | 30.00 |
| Polyvinylpovidone | 14,50 |
| Lactose monohydrate | 77.10 |
| Ethyl cellulose | 24.00 |
| Stearyl alcohol | 59.00 |
| Talc | 5.00 |
| Mg-Stearate | 2.50 |
| Water content of API | 5.7 |
| | |
| Core weight | 277.80 |
| | |
| Opadry II red 85F250018 | 8.00 |
| Talc | 0.61 |
| | |
| Final tablet weight | 286.41 |

| | |
|---|---|
| The amounts indicated refer to the amounts of solids used. API = active pharmaceutical ingredient | |

The tablets were manufactured as described in example 8 and coated as described in example 3.

Tablets OXN 336/10 were then analysed as regards in vitro release behavior using the Ph. European paddle method at 50 rpm in 500 ml simulated gastric fluid (SGFₛₚ) dissolution medium. Aliquots of the dissolution media are withdrawn at the respective time points and analysed by HPLC at 230 nm.

The in vitro release data is indicated as percentage (based on the label content of active tested) in table 35. The upper and lower limits of the desired *in vitro* release range are indicated.

**Table 35**

| | | | Tablets | OXN 336/10 | |
|---|---|---|---|---|---|
| | | | Dissolution medium | SGFₛₚ | |
| time (min) | min. limit | max. limit | | Oxy | Nal |
| 15 | 10% | 30% | | 17% | 17% |
| 120 | 40% | 60% | | 49% | 50% |
| 600 | 80% | | | 94% | 94% |

| | | | | | |
|---|---|---|---|---|---|
| Oxy = oxycodone HCl, Nal = naloxone HCl. min. limit: indicates the lower limit of the amount of oxycodone that should be released at this point in time, max. limit: indicates the upper limit of the amount of oxycodone that should be released at this point in time | | | | | |

Tablets OXN 336/10 were then stored for 3 and 6 months at 25°C and 60% rH, for 3, and 6 months at 30°C and 75% rH or for 3 and 6 months at 40°C and 75% rH. Subsequently, OXN 336/10 were analysed as regards in vitro release behavior using the Ph. European paddle method at 50 rpm in 500 ml simulated gastric fluid (SGFₛₚ) dissolution medium. Aliquots of the dissolution media are withdrawn at the respective time points and analysed by HPLC at 230 nm.

The in vitro release data is indicated as percentage (based on the label content of active tested) in tables 36, 37 and 38 for 6 months of storage to allow for a comparison. The upper and lower limits of the desired *in vitro* release range are indicated.

**Table 36**

| | | | Tablets | OXN 336/10 | | OXN 336/10 | |
|---|---|---|---|---|---|---|---|
| | | | Dissolution medium | SGFₛₚ | | SGFₛₚ | |
| | | | Storage condition | Initial | | 6 months@25°C, 60%rH | |
| time (min) | min. limit | max. limit | | Oxy | Nal | Oxy | Nal |
| 15 | 10% | 30% | | 17% | 17% | 19% | 19% |
| 120 | 40% | 60% | | 49% | 50% | 52% | 52% |
| 600 | 80% | | | 94% | 94% | 95% | 95% |

| | | | | | | | |
|---|---|---|---|---|---|---|---|
| Oxy = oxycodone HCl, Nal = naloxone HCl. min. limit: indicates the lower limit of the amount of oxycodone that should be released at this point in time, max. limit: indicates the upper limit of the amount of oxycodone that should be released at this point in time | | | | | | | |

**Table 37**

| | | | Tablets | OXN 336/10 | | OXN 336/10 | |
|---|---|---|---|---|---|---|---|
| | | | Dissolution medium | SGFₛₚ | | SGFₛₚ | |
| | | | Storage condition | Initial | | 6 months@30°C, 75%rH | |
| time (min) | min. limit | max. limit | | Oxy | Nal | Oxy | Nal |
| 15 | 10% | 30% | | 17% | 17% | 18% | 18% |
| 120 | 40% | 60% | | 49% | 50% | 53% | 53% |
| 600 | 80% | | | 94% | 94% | 96% | 95% |

| | | | | | | | |
|---|---|---|---|---|---|---|---|
| Oxy = oxycodone HCl, Nal = naloxone HCl. min. limit: indicates the lower limit of the amount of oxycodone that should be released at this point in time, max. limit: indicates the upper limit of the amount of oxycodone that should be released at this point in time | | | | | | | |

**Table 38**

| | | | Tablets | OXN 336/10 | | OXN 336/10 | |
|---|---|---|---|---|---|---|---|
| | | | Dissolution medium | SGFₛₚ | | SGFₛₚ | |
| | | | Storage condition | Initial | | 6 months@40°C, 75%rH | |
| time (min) | min. limit | max. limit | | Oxy | Nal | Oxy | Nal |
| 15 | 10% | 30% | | 17% | 17% | 20% | 19% |
| 120 | 40% | 60% | | 49% | 50% | 57% | 56% |
| 600 | 80% | | | 94% | 94% | 99% | 96% |

| | | | | | | | |
|---|---|---|---|---|---|---|---|
| Oxy = oxycodone HCl, Nal = naloxone HCl. min. limit: indicates the lower limit of the amount of oxycodone that should be released at this point in time, max. limit: indicates the upper limit of the amount of oxycodone that should be released at this point in time | | | | | | | |

The amount of total related substances was measured as described in example 1. The results are shown in Table 39. Again the comparison for 6 months of storage is shown.

**Table 39**

| Tablet | OXN336/10 | OXN336/10 | OXN336/10 | OXN336/10 |
|---|---|---|---|---|
| Storage conditions | Initial | 6months @25°C/60%rH | 6months @30°C/75%rH | 6months @40°C/75%rH |
| Noroxymor phone | 0.04% | 0.09% | 0.13% | 0.23% |
| Total Impurities | 0.1% | 0.2% | 0.5% | 1.5% |

The amount of noroxymorphone was the highest of all know related substances and below 0.23% under any storage conditions tested.

The amount of total impurities was below 1.5% under any storage conditions tested.

### Example 13:

Tablets of the composition as shown in Table 40 were manufactured.

**Table 40**

| Tablet | OXN 347/11 |
|---|---|
| Ingredient | Amount (mg) |
| Oxycodone HCI | 80.00 |
| Naloxone HCI | 40.00 |
| Polyvinylpovidone | 14,50 |
| Lactose monohydrate | 45.20 |
| Ethyl cellulose | 24.00 |
| Stearyl alcohol | 59.00 |
| Talc | 5.00 |
| Mg-Stearate | 2.50 |
| Water content of API | 7.60 |
| | |
| Core weight | 277.80 |
| | |
| Opadry II red 85F250018 | 8.00 |
| Talc | 0.61 |
| | |
| Final tablet weight | 286.41 |

| | |
|---|---|
| The amounts indicated refer to the amounts of solids used. API = active pharmaceutical ingredient | |

The tablets were manufactured as described in example 8 and coated as described in example 3.

Tablets OXN 347/11 were then analysed as regards in vitro release behavior using the Ph. European paddle method at 50 rpm in 500 ml simulated gastric fluid (SGFₛₚ) dissolution medium. Aliquots of the dissolution media are withdrawn at the respective time points and analysed by HPLC at 230 nm.

The in vitro release data is indicated as percentage (based on the label content of active tested) in table 41. The upper and lower limits of the desired *in vitro* release range are indicated.

**Table 41**

| | | | Tablets | OXN 347/11 | |
|---|---|---|---|---|---|
| | | | Dissolution medium | SGFₛₚ | |
| time (min) | min. limit | max. limit | | Oxy | Nal |
| 15 | 10% | 30% | | 19% | 19% |
| 120 | 40% | 60% | | 54% | 54% |
| 600 | 80% | | | 98% | 97% |

| | | | | | |
|---|---|---|---|---|---|
| Oxy = oxycodone HCl, Nal = naloxone HCl. min. limit: indicates the lower limit of the amount of oxycodone that should be released at this point in time, max. limit: indicates the upper limit of the amount of oxycodone that should be released at this point in time | | | | | |

Tablets OXN 347/11 were then stored for 3 months at 25°C and 60% rH, for 3 months at 30°C and 75% rH or for 3 months at 40°C and 75% rH. Subsequently, OXN 347/11 were analysed as regards in vitro release behavior using the Ph. European paddle method at 50 rpm in 500 ml simulated gastric fluid (SGFₛₚ) dissolution medium. Aliquots of the dissolution media are withdrawn at the respective time points and analysed by HPLC at 230 nm.

The in vitro release data is indicated as percentage (based on the label content of active tested) in tables 42, 43 and 44 for 3 months of storage to allow for a comparison. The upper and lower limits of the desired *in vitro* release range are indicated.

**Table 42**

| | | | Tablets | OXN 347/11 | | OXN 347/11 | |
|---|---|---|---|---|---|---|---|
| | | | Dissolution medium | SGFₛₚ | | SGFₛₚ | |
| | | | Storage condition | Initial | | 3 months@25°C, 60%rH | |
| time (min) | min. limit | max. limit | | Oxy | Nal | Oxy | Nal |
| 15 | 10% | 30% | | 19% | 19% | 20% | 20% |
| 120 | 40% | 60% | | 54% | 54% | 55% | 55% |
| 600 | 80% | | | 98% | 97% | 99% | 98% |

| | | | | | | | |
|---|---|---|---|---|---|---|---|
| Oxy = oxycodone HCl, Nal = naloxone HCl. min. limit: indicates the lower limit of the amount of oxycodone that should be released at this point in time, max. limit: indicates the upper limit of the amount of oxycodone that should be released at this point in time | | | | | | | |

**Table 43**

| | | | Tablets | OXN 347/11 | | OXN 347/11 | |
|---|---|---|---|---|---|---|---|
| | | | Dissolution medium | SGFₛₚ | | SGFₛₚ | |
| | | | Storage condition | Initial | | 3 months@30°C, 75%rH | |
| time (min) | min. limit | max. limit | | Oxy | Nal | Oxy | Nal |
| 15 | 10% | 30% | | 19% | 19% | 20% | 20% |
| 120 | 40% | 60% | | 54% | 54% | 57% | 57% |
| 600 | 80% | | | 98% | 97% | 98% | 98% |

| | | | | | | | |
|---|---|---|---|---|---|---|---|
| Oxy = oxycodone HCl, Nal = naloxone HCl. min. limit: indicates the lower limit of the amount of oxycodone that should be released at this point in time, max. limit: indicates the upper limit of the amount of oxycodone that should be released at this point in time | | | | | | | |

**Table 44**

| | | | Tablets | OXN 347/11 | | OXN 347/11 | |
|---|---|---|---|---|---|---|---|
| | | | Dissolution medium | SGFₛₚ | | SGFₛₚ | |
| | | | Storage condition | Initial | | 3 months@40°C, 75%rH | |
| time (min) | min. limit | max. limit | | Oxy | Nal | Oxy | Nal |
| 15 | 10% | 30% | | 19% | 19% | 24% | 23% |
| 120 | 40% | 60% | | 54% | 54% | 63% | 62% |
| 600 | 80% | | | 98% | 97% | 100% | 98% |

| | | | | | | | |
|---|---|---|---|---|---|---|---|
| Oxy = oxycodone HCl, Nal = naloxone HCl. min. limit: indicates the lower limit of the amount of oxycodone that should be released at this point in time, max. limit: indicates the upper limit of the amount of oxycodone that should be released at this point in time | | | | | | | |

The amount of total related substances was measured as described in example 1. The results are shown in Table 45. Again the comparison for 3 months of storage is shown.

**Table 45**

| Tablet | OXN 347/11 | OXN 347/11 | OXN 347/11 | OXN 347/11 |
|---|---|---|---|---|
| storage conditions | | Initial 3 months @25°C/60%rH | 3months @30°C/75%rH | 3months @40°C/75%rH |
| Noroxymor phone | 0.00% | 0.07% | 0.09% | 0.15% |
| Total Impurities | 0.1% | 0.2% | 0.4% | 0.8% |

The amount of noroxymorphone was the highest of all know related substances and below 0.15% under any storage conditions tested.

The amount of total impurities was below 0.8% under any storage conditions tested.

Some embodiments of the invention relate to:
1. Use of a binder for manufacturing a prolonged release pharmaceutical composition comprising oxycodone or a pharmaceutically acceptable salt thereof and naloxone or a pharmaceutically acceptable salt thereof wherein the pharmaceutical composition after storage under stressed conditions has less than 4 % of total substances related to oxycodone or a pharmaceutically acceptable salt or derivative thereof and/or related to naloxone or a pharmaceutically acceptable salt or derivative thereof.
2. Use of a binder for manufacturing a prolonged release pharmaceutical composition comprising oxycodone or a pharmaceutically acceptable salt thereof and naloxone or a pharmaceutically acceptable salt thereof wherein the pharmaceutical composition after storage under stressed conditionspharmaceutical composition has less than 0.5 % of known oxycodone or a pharmaceutically acceptable salt or derivative thereof related substances or known naloxone or a pharmaceutically acceptable salt or derivative thereof related substances.
3. Use of a binder for manufacturing a prolonged release pharmaceutical composition comprising oxycodone or a pharmaceutically acceptable salt thereof and naloxone or a pharmaceutically acceptable salt thereof, wherein the pharmaceutical composition after storage under stressed conditions releases the pharmaceutically active agents with substantially the same release rate as before subjecting the pharmaceutical composition to stressed conditions.
4. Use according to 1, 2 or 3, wherein the pharmaceutical composition comprises at least part of oxycodone or a pharmaceutically acceptable salt or derivative thereof and at least part of naloxone or a pharmaceutically acceptable salt or derivative thereof in a prolonged release matrix.
5. Use according to any of 1 to 4, wherein the pharmaceutical composition comprises oxycodone or a pharmaceutically acceptable salt or derivative thereof in an amount equivalent to 1 to 15 mg, preferably 1 to 9 mg of oxycodone HCl and naloxone or a pharmaceutically acceptable salt or derivative thereof in an amount equivalent to 0.5 to 7.5 mg, preferably 0.5 to 4.5 mg of naloxone HCl.
6. Use according to any of 1 to 5, wherein the pharmaceutical composition comprises hydroxypropyl cellulose as a binder.
7. Use according to any of 5 or 6, wherein the pharmaceutical composition releases the pharmaceutically active agents with the following in vitro release rate when measured using the Ph. Eur. paddle method in 500 ml of Simulated Gastric Fluid at 50 rpm at 37 degrees C°:

| | |
|---|---|
| at 15 min: | 10 to 30 % by weight of the pharmaceutically active agents, |
| at 2 h: | 40 to 60% by weight of the pharmaceutically active agents, |
| at 10 h: | more than 80% by weight of the pharmaceutically active agents. |

8. Use according to any of 1 to 4, wherein the pharmaceutical composition comprises oxycodone or a pharmaceutically acceptable salt or derivative thereof in an amount equivalent to 10 to 160 mg, preferably 16 to 160 mg of oxycodone HCl and naloxone or a pharmaceutically acceptable salt or derivative thereof in an amount equivalent to 5 to 80 mg, preferably 8 to 80 mg of naloxone HCl.
9. Use according to 8, wherein the pharmaceutical composition comprises povidone as a binder.
10. Use according to any of 8 or 9, wherein the composition releases the pharmaceutically active agents with the following in vitro release rate when measured using the Ph. Eur. paddle method in 900 ml of Simulated Gastric Fluid at 50 rpm at 37 degrees C°:

| | |
|---|---|
| at 15 min: | 10 to 30 % by weight of the pharmaceutically active agents, |
| at 2 h: | 40 to 65% by weight of the pharmaceutically active agents, |
| at 10 h: | more than 80% by weight of the pharmaceutically active agents. |

11. Use of HPC for stabilizing a prolonged release pharmaceutical composition comprising oxycodone or a pharmaceutically acceptable salt thereof and naloxone or a pharmaceutically acceptable salt thereof, wherein the pharmaceutical composition comprises at least part of oxycodone or a pharmaceutically acceptable salt thereof and of naloxone or a pharmaceutically acceptable salt thereof in a prolonged release matrix, wherein the pharmaceutical composition comprises oxycodone or a pharmaceutically acceptable salt thereof in an amount equivalent to 1 to 15 mg, preferably 1 to 9 mg of oxycodone HCl and naloxone or a pharmaceutically acceptable salt thereof in an amount equivalent to 0.5 to 7.5 mg, preferably 0.5 to 4.5 mg of naloxone HCl and wherein the pharmaceutical composition comprises oxycodone or a pharmaceutically acceptable salt thereof and naloxone or a pharmaceutically acceptable salt thereof in 2:1 ratio by weight.
12. An oral prolonged release pharmaceutical composition comprising at least:
a) at least one prolonged release material;
b) at least oxycodone or a pharmaceutically acceptable salt or derivative thereof and naloxone or a pharmaceutically acceptable salt or derivative thereof; and wherein
c) the pharmaceutical composition after storage under stressed conditions has less than 4 % of total substances related to oxycodone or a pharmaceutically acceptable salt or derivative thereof and/or related to naloxone or a pharmaceutically acceptable salt or derivative thereof.
13. An oral prolonged release pharmaceutical composition comprising at least:
a) at least one prolonged release material;
b) at least oxycodone or a pharmaceutically acceptable salt or derivative thereof and naloxone or a pharmaceutically acceptable salt or derivative thereof; and wherein
c) the pharmaceutical composition after storage under stressed conditions has less than 0.5 % of known oxycodone or a pharmaceutically acceptable salt or derivative thereof related substances or known naloxone or a pharmaceutically acceptable salt or derivative thereof related substances.
14. An oral prolonged release pharmaceutical composition, comprising at least:
a) at least one prolonged release material;
b) at least oxycodone or a pharmaceutically acceptable salt or derivative thereof and naloxone or a pharmaceutically acceptable salt or derivative thereof; and wherein the
c) pharmaceutical composition after storage under stressed conditions releases the pharmaceutically active agents with substantially the same release rate as before subjecting the pharmaceutical composition to stressed conditions.
15. Pharmaceutical composition according to 12, 13 or 14, wherein the prolonged release pharmaceutical composition comprises oxycodone or a pharmaceutically acceptable salt or derivative thereof in an amount equivalent to 1 to 15 mg, preferably 1 to 9 mg of oxycodone HCl and naloxone or a pharmaceutically acceptable salt or derivative thereof in an amount equivalent to 0.5 to 7.5 mg, preferably 0.5 to 4.5 mg of naloxone HCl and wherein the at least one prolonged release material and oxycodone or a pharmaceutically acceptable salt or derivative thereof and naloxone or a pharmaceutically acceptable salt or derivative thereof are combined to form a prolonged release matrix.
16. Pharmaceutical composition according to any of 12 to 15 wherein the pharmaceutical composition comprises additionally at least one filler, at least one lubricant and at least one binder.
17. Pharmaceutical composition according to 16, wherein the prolonged release pharmaceutical composition comprises hydroxypropyl cellulose as a binder.
18. Pharmaceutical composition according to any of 12 to 17, wherein the composition releases the pharmaceutically active agents with the following in vitro release rate when measured using the Ph. Eur. paddle method in 500 ml of Simulated Gastric Fluid at 50 rpm at 37 degrees C°:

| | |
|---|---|
| at 15 min: | 10 to 30 % by weight of the pharmaceutically active agents, |
| at 2 h: | 40 to 60% by weight of the pharmaceutically active agents, |
| at 10 h: | more than 80% by weight of the pharmaceutically active agents. |

19. Pharmaceutical composition according to 12, 13 or 14, wherein the prolonged release pharmaceutical composition comprises oxycodone or a pharmaceutically acceptable salt or derivative thereof in an amount equivalent to 10 to 160 mg, preferably 16 to 160 mg of oxycodone HCl and naloxone or a pharmaceutically acceptable salt or derivative thereof in an amount equivalent to 5 to 80 mg, preferably 8 to 80 mg of naloxone HCl and wherein the at least one prolonged release material and oxycodone or a pharmaceutically acceptable salt or derivative thereof and naloxone or a pharmaceutically acceptable salt or derivative thereof are combined to form a prolonged release matrix.
20. Pharmaceutical composition according to any of 12, 13, 14, or 19, wherein the pharmaceutical composition comprises additionally at least one filler, at least one lubricant and at least one binder.
21. Pharmaceutical composition according to 20, wherein the prolonged release pharmaceutical composition does not comprise povidone as a binder.
22. Pharmaceutical composition according to any of 19 to 22, wherein the composition releases the pharmaceutically active agents with the following in vitro release rate when measured using the Ph. Eur. paddle method in 900 ml of Simulated Gastric Fluid at 50 rpm at 37 degrees C°:

| | |
|---|---|
| at 15 min: | 10 to 30 % by weight of the pharmaceutically active agents, |
| at 2 h: | 40 to 65% by weight of the pharmaceutically active agents, |
| at 10 h: | more than 80% by weight of the pharmaceutically active agents. |

23. Pharmaceutical dosage form according to any of 12 to 22, wherein oxycodone hydrochloride and naloxone hydrochloride are used.

Some further embodiments of the invention relate to:
1. An oral prolonged release pharmaceutical composition comprising at least:
a) at least one prolonged release material;
b) at least oxycodone or a pharmaceutically acceptable salt or derivative thereof and naloxone or a pharmaceutically acceptable salt or derivative thereof; and wherein
c) the pharmaceutical composition after storage under stressed conditions has less than 4 % of total substances related to oxycodone or a pharmaceutically acceptable salt or derivative thereof and/or related to naloxone or a pharmaceutically acceptable salt or derivative thereof.
2. An oral prolonged release pharmaceutical composition comprising at least:
a) at least one prolonged release material;
b) at least oxycodone or a pharmaceutically acceptable salt or derivative thereof and naloxone or a pharmaceutically acceptable salt or derivative thereof; and wherein
c) the pharmaceutical composition after storage under stressed conditions has less than 0.5 % of known oxycodone or a pharmaceutically acceptable salt or derivative thereof related substances or known naloxone or a pharmaceutically acceptable salt or derivative thereof related substances.
3. Pharmaceutical composition according to embodiment 1 or 2, wherein the prolonged release pharmaceutical composition comprises oxycodone or a pharmaceutically acceptable salt thereof in an amount equivalent to 1 to 15 mg, preferably 1 to 9 mg of oxycodone HCl and naloxone or a pharmaceutically acceptable salt thereof in an amount equivalent to 0.5 to 7.5 mg, preferably 0.5 to 4.5 mg of naloxone HCl.
4. Pharmaceutical composition according to embodiment 1, 2 or 3, wherein the prolonged release pharmaceutical composition comprises oxycodone or a pharmaceutically acceptable salt thereof and naloxone or a pharmaceutically acceptable salt thereof in a ratio of 2:1 by weight.
5. Pharmaceutical composition according to embodiment 1, 2, 3 or 4, wherein the prolonged release pharmaceutical composition comprises oxycodone or a pharmaceutically acceptable salt thereof in an amount equivalent to 1 mg of oxycodone HCl and naloxone or a pharmaceutically acceptable salt thereof in an amount equivalent to 0.5 mg of naloxone HCl, oxycodone or a pharmaceutically acceptable salt thereof in an amount equivalent to 2.5 mg of oxycodone HCl and naloxone or a pharmaceutically acceptable salt thereof in an amount equivalent to 1.25 mg of naloxone HCl, oxycodone or a pharmaceutically acceptable salt thereof in an amount equivalent to 5 mg of oxycodone HCl and naloxone or a pharmaceutically acceptable salt thereof in an amount equivalent to 2.5 mg of naloxone HCl, oxycodone or a pharmaceutically acceptable salt thereof in an amount equivalent to 7.5 mg of oxycodone HCl and naloxone in an amount equivalent to 3.75 mg of naloxone HCl, oxycodone or a pharmaceutically acceptable salt thereof in an amount equivalent to 10 mg of oxycodone HCl and naloxone in an amount equivalent to 5 mg of naloxone HCl, oxycodone or a pharmaceutically acceptable salt thereof in an amount equivalent to 12.5 mg of oxycodone HCl and naloxone in an amount equivalent to 6.25 mg of naloxone HCl or oxycodone or a pharmaceutically acceptable salt thereof in an amount equivalent to 15 mg of oxycodone HCl and naloxone in an amount equivalent to 7.5 mg of naloxone HCl.
6. Pharmaceutical composition according to any of embodiments 1 to 5, wherein the at least one prolonged release material and oxycodone or a pharmaceutically acceptable salt thereof and naloxone or a pharmaceutically acceptable salt thereof are combined to form a prolonged release matrix.
7. Pharmaceutical composition according to embodiment 6, wherein the prolonged release material is selected from the group comprising hydrophobic or hydrophilic polymers, gums, substituted or unsubstituted hydrocarbons, carbohydrates, fatty acids, fatty alcohols, glyceryl esters of fatty acids, natural and synthetic oils and waxes.
8. Pharmaceutical composition according to embodiment 7, wherein the prolonged release material is a cellulose ether, a (meth)acrylic based (co)polymer and/or a fatty alcohol.
9. Pharmaceutical composition according to any of embodiments 6, 7 or 8, comprising at least:
a) at least one cellulose ether, preferably at least one hydrophobic cellulose ether such as ethyl cellulose as prolonged release material;
b) at least oxycodone or a pharmaceutically acceptable salt or derivative thereof and naloxone or a pharmaceutically acceptable salt or derivative thereof, wherein
c) oxycodone or a pharmaceutically acceptable salt or derivative thereof and naloxone or a pharmaceutically acceptable salt or derivative thereof are combined with said prolonged release material to form a prolonged release matrix.
10. Pharmaceutical composition according to any of embodiments 6, 7 or 8, comprising at least:
a) at least one fatty alcohol as prolonged release material;
b) at least oxycodone or a pharmaceutically acceptable salt or derivative thereof and naloxone or a pharmaceutically acceptable salt or derivative thereof, wherein
c) oxycodone or a pharmaceutically acceptable salt or derivative thereof and naloxone or a pharmaceutically acceptable salt or derivative thereof are combined with said prolonged release material to form a prolonged release matrix.
11. Pharmaceutical composition according to any of embodiments 6, 7 or 8, comprising at least:
a) at least one cellulose ether, preferably at least one hydrophobic cellulose ether such as ethyl cellulose and at least one fatty alcohol as prolonged release materials;
b) at least oxycodone or a pharmaceutically acceptable salt or derivative thereof and naloxone or a pharmaceutically acceptable salt or derivative thereof, wherein
c) oxycodone or a pharmaceutically acceptable salt or derivative thereof and naloxone or a pharmaceutically acceptable salt or derivative thereof are combined with said prolonged release materials to form a prolonged release matrix.
12. Pharmaceutical composition according to any of embodiments 6, 7, 8, 9, 10 or 11 wherein the pharmaceutical composition comprises additionally at least one filler, at least one lubricant and at least one binder.
13. Pharmaceutical composition according to embodiment 12, wherein said filler is lactose monohydrate.
14. Pharmaceutical composition according to embodiment 12, wherein magnesium stearate and/or talc are used as lubricants.
15. Pharmaceutical composition according to embodiment 12, wherein hydroxypropyl cellulose, povidone, co-povidone, hydroxypropylmethyl cellulose polyvinyl pyrrolidone vinyl alcohol (PVPVA), microcrystalline cellulose, polyethylene glycols (PEGs), gelatine, starch or glycerol esters of fatty acids is used as binder.
16. Pharmaceutical composition according to embodiment 12, wherein the prolonged release pharmaceutical composition does not comprise hydroxypropyl cellulose as a binder.
17. Pharmaceutical composition according to embodiment 12, wherein the prolonged release pharmaceutical composition comprises hydroxypropyl cellulose as a binder.
18. Pharmaceutical composition according to any of embodiments 1 to 17, wherein the composition releases the pharmaceutically active agents with the following in vitro release rate when measured using the Ph. Eur. paddle method in 500 ml of Simulated Gastric Fluid at 50 rpm at 37 degrees C°:

| | |
|---|---|
| at 15 min: | 10 to 30 % by weight of the pharmaceutically active agents, |
| at 2 h: | 40 to 60% by weight of the pharmaceutically active agents, |
| at 10 h: | more than 80% by weight of the pharmaceutically active agents. |

19. Pharmaceutical composition according to embodiment 1 or 2, wherein the prolonged release pharmaceutical composition comprises oxycodone or a pharmaceutically acceptable salt thereof in an amount equivalent to 10 to 160 mg, preferably 16 to 160 mg of oxycodone HCl and naloxone or a pharmaceutically acceptable salt thereof in an amount equivalent to 5 to 80, preferably 8 to 80 mg of naloxone HCl.
20. Pharmaceutical composition according to embodiment 1, 2 or 19, wherein the prolonged release pharmaceutical composition comprises oxycodone or a pharmaceutically acceptable salt thereof and naloxone or a pharmaceutically acceptable salt thereof in a ratio of 2:1 by weight.
21. Pharmaceutical composition according to any of embodiments 1, 2, 19 or 20, wherein the prolonged release pharmaceutical composition comprises oxycodone or a pharmaceutically acceptable salt thereof in an amount equivalent to 10 mg of oxycodone HCl and naloxone or a pharmaceutically acceptable salt thereof in an amount equivalent to 5 mg of naloxone HCl, oxycodone or a pharmaceutically acceptable salt thereof in an amount equivalent to 20 mg of oxycodone HCl and naloxone or a pharmaceutically acceptable salt thereof in an amount equivalent to 10 mg of naloxone HCl, oxycodone or a pharmaceutically acceptable salt thereof in an amount equivalent to 30 mg of oxycodone HCl and naloxone or a pharmaceutically acceptable salt thereof in an amount equivalent to 15 mg of naloxone HCl, oxycodone or a pharmaceutically acceptable salt thereof in an amount equivalent to 40 mg of oxycodone HCl and naloxone or a pharmaceutically acceptable salt thereof in an amount equivalent to 20 mg of naloxone HCl, oxycodone or a pharmaceutically acceptable salt thereof in an amount equivalent to 60 mg of oxycodone HCl and naloxone or a pharmaceutically acceptable salt thereof in an amount equivalent to 30 mg of naloxone HCl, oxycodone or a pharmaceutically acceptable salt thereof in an amount equivalent to 80 mg of oxycodone HCl and naloxone or a pharmaceutically acceptable salt thereof in an amount equivalent to 40 mg of naloxone HCl and oxycodone or a pharmaceutically acceptable salt thereof in an amount equivalent to 160 mg of oxycodone HCl and naloxone or a pharmaceutically acceptable salt thereof in an amount equivalent to 80 mg of naloxone HCl.
22. Pharmaceutical composition according to any of embodiments 1, 2, 19, 20 or 21, wherein the at least one prolonged release material and oxycodone or a pharmaceutically acceptable salt thereof and naloxone or a pharmaceutically acceptable salt thereof are combined to form a prolonged release matrix.
23. Pharmaceutical composition according to embodiment 22, wherein the prolonged release material is selected from the group comprising hydrophobic or hydrophilic polymers, gums, substituted or unsubstituted hydrocarbons, carbohydrates, fatty acids, fatty alcohols, glyceryl esters of fatty acids, natural and synthetic oils and waxes.
24. Pharmaceutical composition according to embodiment 22, wherein the prolonged release material is a cellulose ether, a (meth)acrylic based (co)polymer and/or a fatty alcohol.
25. Pharmaceutical composition according to any of embodiments 22, 23 or 24, comprising at least:
a) at least one cellulose ether, preferably at least one hydrophobic cellulose ether such as ethyl cellulose as prolonged release material;
b) at least oxycodone or a pharmaceutically acceptable salt or derivative thereof and naloxone or a pharmaceutically acceptable salt or derivative thereof, wherein
c) oxycodone or a pharmaceutically acceptable salt or derivative thereof and naloxone or a pharmaceutically acceptable salt or derivative thereof are combined with said prolonged release material to form a prolonged release matrix.
26. Pharmaceutical composition according to any of embodiments 22, 23 or 24, comprising at least:
a) at least one fatty alcohol as prolonged release material;
b) at least oxycodone or a pharmaceutically acceptable salt or derivative thereof and naloxone or a pharmaceutically acceptable salt or derivative thereof, wherein
c) oxycodone or a pharmaceutically acceptable salt or derivative thereof and naloxone or a pharmaceutically acceptable salt or derivative thereof are combined with said prolonged release material to form a prolonged release matrix.
27. Pharmaceutical composition according to any of embodiments 22, 23 or 24, comprising at least:
a) at least one cellulose ether, preferably at least one hydrophobic cellulose ether such as ethyl cellulose and at least one fatty alcohol as prolonged release materials;
b) at least oxycodone or a pharmaceutically acceptable salt or derivative thereof and naloxone or a pharmaceutically acceptable salt or derivative thereof, wherein
c) oxycodone or a pharmaceutically acceptable salt or derivative thereof and naloxone or a pharmaceutically acceptable salt or derivative thereof are combined with said prolonged release materials to form a prolonged release matrix.
28. Pharmaceutical composition according to any of embodiments 22, 23, 24, 25, 26 or 27, wherein the pharmaceutical composition comprises additionally at least one filler, at least one lubricant and at least one binder.
29. Pharmaceutical composition according to embodiment 28, wherein said filler is lactose monohydrate.
30. Pharmaceutical composition according to embodiment 28, wherein magnesium stearate and/or talc are used as lubricants.
31. Pharmaceutical composition according to embodiment 28, wherein co-povidone, hydroxypropylmethyl cellulose, hydroxypropyl cellulose, polyvinyl pyrrolidone vinyl alcohol (PVPVA), microcrystalline cellulose, polyethylene glycols (PEGs), gelatine, starch or glycerol esters of fatty acids is used as binder.
32. Pharmaceutical composition according to embodiment 28, wherein the prolonged release pharmaceutical composition does not comprise povidone as a binder.
33. Pharmaceutical composition according to any of embodiments 19 to 32, wherein the composition releases the pharmaceutically active agents with the following in vitro release rate when measured using the Ph. Eur. paddle method in 900 ml of Simulated Gastric Fluid at 50 rpm at 37 degrees C°:

| | |
|---|---|
| at 15 min: | 10 to 30 % by weight of the pharmaceutically active agents, |
| at 2 h: | 40 to 65% by weight of the pharmaceutically active agents, |
| at 10 h: | more than 80% by weight of the pharmaceutically active agents. |

34. An oral prolonged release pharmaceutical composition, comprising at least:
a) at least one prolonged release material;
b) at least oxycodone or a pharmaceutically acceptable salt or derivative thereof and naloxone or a pharmaceutically acceptable salt or derivative thereof; and wherein the
c) pharmaceutical composition after storage under stressed conditions releases the pharmaceutically active agents with substantially the same release rate as before subjecting the pharmaceutical composition to stressed conditions.
35. Pharmaceutical composition according to embodiment 34, wherein the prolonged release pharmaceutical composition comprises oxycodone or a pharmaceutically acceptable salt thereof in an amount equivalent to 1 to 15 mg, preferably 1 to 9 mg of oxycodone HCl and naloxone or a pharmaceutically acceptable salt thereof in an amount equivalent to 0.5 to 7.5 mg, preferably 0.5 to 4.5 mg of naloxone HCl.
36. Pharmaceutical composition according to 34 or 35, wherein the prolonged release pharmaceutical composition comprises oxycodone or a pharmaceutically acceptable salt thereof and naloxone or a pharmaceutically acceptable salt thereof in a ratio of 2:1 by weight.
37. Pharmaceutical composition according to embodiment 34, 35 or 36, wherein the prolonged release pharmaceutical composition comprises oxycodone or a pharmaceutically acceptable salt thereof in an amount equivalent to 1 mg of oxycodone HCl and naloxone or a pharmaceutically acceptable salt thereof in an amount equivalent to 0.5 mg of naloxone HCl, oxycodone or a pharmaceutically acceptable salt thereof in an amount equivalent to 2.5 mg of oxycodone HCl and naloxone or a pharmaceutically acceptable salt thereof in an amount equivalent to 1.25 mg of naloxone HCl, oxycodone or a pharmaceutically acceptable salt thereof in an amount equivalent to 5 mg of oxycodone HCl and naloxone or a pharmaceutically acceptable salt thereof in an amount equivalent to 2.5 mg of naloxone HCl, oxycodone or a pharmaceutically acceptable salt thereof in an amount equivalent to 7.5 mg of oxycodone HCl and naloxone or a pharmaceutically acceptable salt thereof in an amount equivalent to 3.75 mg of naloxone HCl, oxycodone or a pharmaceutically acceptable salt thereof in an amount equivalent to 10 mg of oxycodone HCl and naloxone or a pharmaceutically acceptable salt thereof in an amount equivalent to 5 mg of naloxone HCl, oxycodone or a pharmaceutically acceptable salt thereof in an amount equivalent to 12.5 mg of oxycodone HCl and naloxone or a pharmaceutically acceptable salt thereof in an amount equivalent to 6.25 mg of naloxone HCl or oxycodone or a pharmaceutically acceptable salt thereof in an amount equivalent to 15 mg of oxycodone HCl and naloxone or a pharmaceutically acceptable salt thereof in an amount equivalent to 7.5 mg of naloxone HCl.
38. Pharmaceutical composition according to any of embodiments 34 to 37, wherein the at least one prolonged release material and oxycodone or a pharmaceutically acceptable salt thereof and naloxone or a pharmaceutically acceptable salt thereof are combined to form a prolonged release matrix.
39. Pharmaceutical composition according to embodiment 38, wherein the prolonged release material is selected from the group comprising hydrophobic or hydrophilic polymers, gums, substituted or unsubstituted hydrocarbons, carbohydrates, fatty acids, fatty alcohols, glyceryl esters of fatty acids, natural and synthetic oils and waxes.
40. Pharmaceutical composition according to embodiment 39, wherein the prolonged release material is a cellulose ether, a (meth)acrylic based (co)polymer and/or a fatty alcohol.
41. Pharmaceutical composition according to any of embodiments 38, 39 or 40, comprising at least:
a) at least one cellulose ether, preferably at least one hydrophobic cellulose ether such as ethyl cellulose as prolonged release material;
b) at least oxycodone or a pharmaceutically acceptable salt or derivative thereof and naloxone or a pharmaceutically acceptable salt or derivative thereof, wherein
c) oxycodone or a pharmaceutically acceptable salt or derivative thereof and naloxone or a pharmaceutically acceptable salt or derivative thereof are combined with said prolonged release material to form a prolonged release matrix.
42. Pharmaceutical composition according to any of embodiments 38, 39 or 40, comprising at least:
a) at least one fatty alcohol as prolonged release material;
b) at least oxycodone or a pharmaceutically acceptable salt or derivative thereof and naloxone or a pharmaceutically acceptable salt or derivative thereof, wherein
c) oxycodone or a pharmaceutically acceptable salt or derivative thereof and naloxone or a pharmaceutically acceptable salt or derivative thereof are combined with said prolonged release material to form a prolonged release matrix.
43. Pharmaceutical composition according to any of embodiments 38, 39 or 40, comprising at least:
a) at least one cellulose ether, preferably at least one hydrophobic cellulose ether such as ethyl cellulose and at least one fatty alcohol as prolonged release materials;
b) at least oxycodone or a pharmaceutically acceptable salt or derivative thereof and naloxone or a pharmaceutically acceptable salt or derivative thereof, wherein
c) oxycodone or a pharmaceutically acceptable salt or derivative thereof and naloxone or a pharmaceutically acceptable salt or derivative thereof are combined with said prolonged release materials to form a prolonged release matrix.
44. Pharmaceutical composition according to any of embodiments 38, 39, 40, 41, 42 or 43 wherein the pharmaceutical composition comprises additionally at least one filler, at least one lubricant and at least one binder.
45. Pharmaceutical composition according to embodiment 44, wherein said filler is lactose monohydrate.
46. Pharmaceutical composition according to embodiment 44, wherein magnesium stearate and/or talc are used as lubricants.
47. Pharmaceutical composition according to embodiment 44, wherein hydroxypropyl cellulose, povidone, co-povidone, hydroxypropylmethyl cellulose polyvinyl pyrrolidone vinyl alcohol (PVPVA), microcrystalline cellulose, polyethylene glycols (PEGs), gelatine, starch or glycerol esters of fatty acids is used as binder.
48. Pharmaceutical composition according to embodiment 44, wherein the prolonged release pharmaceutical composition does not comprise hydroxypropyl cellulose as a binder.
49. Pharmaceutical composition according to embodiment 44, wherein the prolonged release pharmaceutical composition comprises hydroxypropyl cellulose as a binder.
50. Pharmaceutical composition according to any of embodiments 34 to 49, wherein the composition releases the pharmaceutically active agents with the following in vitro release rate when measured using the Ph. Eur. paddle method in 500 ml of Simulated Gastric Fluid at 50 rpm at 37 degrees° C:

| | |
|---|---|
| at 15 min: | 10 to 30 % by weight of the pharmaceutically active agents, |
| at 2 h: | 40 to 60% by weight of the pharmaceutically active agents, |
| at 10 h: | more than 80% by weight of the pharmaceutically active agents. |

51. Pharmaceutical composition according to embodiment 34, wherein the prolonged release pharmaceutical composition comprises oxycodone or a pharmaceutically acceptable salt thereof in an amount equivalent to 10 to 160 mg, preferably 16 to 160 mg of oxycodone HCl and naloxone or a pharmaceutically acceptable salt thereof in an amount equivalent to 5 to 80 mg, preferably 8 to 80 mg of naloxone HCl.
52. Pharmaceutical composition according to embodiment 34 or 51, wherein the prolonged release pharmaceutical composition comprises oxycodone or a pharmaceutically acceptable salt thereof and naloxone or a pharmaceutically acceptable salt thereof in a ratio of 2:1 by weight.
53. Pharmaceutical composition according to any of embodiments 34, 51 or 52, wherein the prolonged release pharmaceutical composition comprises oxycodone or a pharmaceutically acceptable salt thereof in an amount equivalent to 10 mg of oxycodone HCl and naloxone or a pharmaceutically acceptable salt thereof in an amount equivalent to 5 mg of naloxone HCl, oxycodone or a pharmaceutically acceptable salt thereof in an amount equivalent to 20 mg of oxycodone HCl and naloxone or a pharmaceutically acceptable salt thereof in an amount equivalent to 10 mg of naloxone HCl, oxycodone or a pharmaceutically acceptable salt thereof in an amount equivalent to 30 mg of oxycodone HCl and naloxone or a pharmaceutically acceptable salt thereof in an amount equivalent to 15 mg of naloxone HCl, oxycodone or a pharmaceutically acceptable salt thereof in an amount equivalent to 40 mg of oxycodone HCl and naloxone or a pharmaceutically acceptable salt thereof in an amount equivalent to 20 mg of naloxone HCl, oxycodone or a pharmaceutically acceptable salt thereof in an amount equivalent to 60 mg of oxycodone HCl and naloxone or a pharmaceutically acceptable salt thereof in an amount equivalent to 30 mg of naloxone HCl, oxycodone or a pharmaceutically acceptable salt thereof in an amount equivalent to 80 mg of oxycodone HCl and naloxone or a pharmaceutically acceptable salt thereof in an amount equivalent to 40 mg of naloxone HCl and oxycodone or a pharmaceutically acceptable salt thereof in an amount equivalent to 160 mg of oxycodone HCl and naloxone or a pharmaceutically acceptable salt thereof in an amount equivalent to 80 mg of naloxone HCl.
54. Pharmaceutical composition according to any of embodiments 34, 51, 52 or 53, wherein the at least one prolonged release material and oxycodone or a pharmaceutically acceptable salt thereof and naloxone or a pharmaceutically acceptable salt thereof are combined to form a prolonged release matrix.
55. Pharmaceutical composition according to embodiment 54, wherein the prolonged release material is selected from the group comprising hydrophobic or hydrophilic polymers, gums, substituted or unsubstituted hydrocarbons, carbohydrates, fatty acids, fatty alcohols, glyceryl esters of fatty acids, natural and synthetic oils and waxes.
56. Pharmaceutical composition according to embodiment 55, wherein the prolonged release material is a cellulose ether, a (meth)acrylic based (co)polymer and/or a fatty alcohol.
57. Pharmaceutical composition according to any of embodiments 54, 55 or 56, comprising at least:
a) at least one cellulose ether, preferably at least one hydrophobic cellulose ether such as ethyl cellulose as prolonged release material;
b) at least oxycodone or a pharmaceutically acceptable salt or derivative thereof and naloxone or a pharmaceutically acceptable salt or derivative thereof, wherein
c) oxycodone or a pharmaceutically acceptable salt or derivative thereof and naloxone or a pharmaceutically acceptable salt or derivative thereof are combined with said prolonged release material to form a prolonged release matrix.
58. Pharmaceutical composition according to any of embodiments 54, 55 or 56, comprising at least:
a) at least one fatty alcohol as prolonged release material;
b) at least oxycodone or a pharmaceutically acceptable salt or derivative thereof and naloxone or a pharmaceutically acceptable salt or derivative thereof, wherein
c) oxycodone or a pharmaceutically acceptable salt or derivative thereof and naloxone or a pharmaceutically acceptable salt or derivative thereof are combined with said prolonged release material to form a prolonged release matrix.
59. Pharmaceutical composition according to any of embodiments 54, 55 or 56, comprising at least:
a) at least one cellulose ether, preferably at least one hydrophobic cellulose ether such as ethyl cellulose and at least one fatty alcohol as prolonged release materials;
b) at least oxycodone or a pharmaceutically acceptable salt or derivative thereof and naloxone or a pharmaceutically acceptable salt or derivative thereof, wherein
c) oxycodone or a pharmaceutically acceptable salt or derivative thereof and naloxone or a pharmaceutically acceptable salt or derivative thereof are combined with said prolonged release materials to form a prolonged release matrix.
60. Pharmaceutical composition according to any of embodiments 54, 55, 56, 57, 58 or 59 wherein the pharmaceutical composition comprises additionally at least one filler, at least one lubricant and at least one binder.
61. Pharmaceutical composition according to embodiment 60, wherein said filler is lactose monohydrate.
62. Pharmaceutical composition according to embodiment 60, wherein magnesium stearate and/or talc are used as lubricants.
63. Pharmaceutical composition according to embodiment 60, wherein co-povidone, hydroxypropylmethyl cellulose, hydroxypropyl cellulose, polyvinyl pyrrolidone vinyl alcohol (PVPVA), microcrystalline cellulose, polyethylene glycols (PEGs), gelatine, starch or glycerol esters of fatty acids is used as binder.
64. Pharmaceutical composition according to embodiment 60, wherein the prolonged release pharmaceutical composition does not comprise povidone as a binder.
65. Pharmaceutical composition according to any of embodiments 34 and 51 to 64, wherein the composition releases the pharmaceutically active agents with the following in vitro release rate when measured using the Ph. Eur. paddle method in 900 ml of Simulated Gastric Fluid at 50 rpm at 37 degrees C°:

| | |
|---|---|
| at 15 min: | 10 to 30 % by weight of the pharmaceutically active agents, |
| at 2 h: | 40 to 65% by weight of the pharmaceutically active agents, |
| at 10 h: | more than 80% by weight of the pharmaceutically active agents. |

66. Pharmaceutical dosage form according to any of embodiments 1 to 65, wherein oxycodone hydrochloride and naloxone hydrochloride are used.
67. Use of a binder for manufacturing a prolonged release pharmaceutical composition comprising oxycodone or a pharmaceutically acceptable salt thereof and naloxone or a pharmaceutically acceptable salt thereof wherein the pharmaceutical composition after storage under stressed conditions has less than 4 % of total substances related to oxycodone or a pharmaceutically acceptable salt or derivative thereof and/or related to naloxone or a pharmaceutically acceptable salt or derivative thereof.
68. Use of a binder for manufacturing a prolonged release pharmaceutical composition comprising oxycodone or a pharmaceutically acceptable salt thereof and naloxone or a pharmaceutically acceptable salt thereof wherein the pharmaceutical composition after storage under stressed conditionspharmaceutical composition has less than 0.5 % of known oxycodone or a pharmaceutically acceptable salt or derivative thereof related substances or known naloxone or a pharmaceutically acceptable salt or derivative thereof related substances.
69. Use of a binder for manufacturing a prolonged release pharmaceutical composition comprising oxycodone or a pharmaceutically acceptable salt thereof and naloxone or a pharmaceutically acceptable salt thereof, wherein the pharmaceutical composition after storage under stressed conditions releases the pharmaceutically active agents with substantially the same release rate as before subjecting the pharmaceutical composition to stressed conditions.
70. Use according to embodiment 67, 68 or 69, wherein the pharmaceutical composition comprises at least part of oxycodone or a pharmaceutically acceptable salt thereof and at least part of naloxone or a pharmaceutically acceptable salt in a prolonged release matrix.
71. Use according to embodiment 70, wherein the prolonged release matrix comprises a prolonged release matrix material being selected from the group comprising hydrophobic or hydrophilic polymers, gums, substituted or unsubstituted hydrocarbons, carbohydrates, fatty acids, fatty alcohols, glyceryl esters of fatty acids, oils and waxes.
72. Use according to embodiment 71, wherein the pharmaceutical composition does not comprise ethyl cellulose and/or stearyl alcohol as prolonged release matrix material.
73. Use according to any of embodiments 67 to 72, wherein the pharmaceutical composition comprises additionally at least one filler and at least one lubricant.
74. Use according to embodiment 73, wherein said filler is lactose monohydrate.
75. Use according to embodiment 73, wherein magnesium stearate and/or talc are used as lubricants.
76. Use according to any of embodiments 67 to 75, wherein the pharmaceutical composition comprises oxycodone or a pharmaceutically acceptable salt thereof in an amount equivalent to 1 to 15 mg, preferably 1 to 9 mg of oxycodone HCl and naloxone or a pharmaceutically acceptable salt thereof in an amount equivalent to 0.5 to 7.5 mg, preferably 0.5 to 4.5 mg of naloxone HCl.
77. Use according to embodiment 76, wherein the pharmaceutical composition comprises oxycodone or a pharmaceutically acceptable salt thereof and naloxone or a pharmaceutically acceptable salt thereof in 2:1 ratio by weight.
78. Use according to embodiment 76 or 77, wherein the pharmaceutical composition comprises oxycodone or a pharmaceutically acceptable salt thereof in an amount equivalent to 1 mg of oxycodone HCl and naloxone or a pharmaceutically acceptable salt thereof in an amount equivalent to 0.5 mg of naloxone HCl, oxycodone or a pharmaceutically acceptable salt thereof in an amount equivalent to 2.5 mg of oxycodone HCl and naloxone or a pharmaceutically acceptable salt thereof in an amount equivalent to 1.25 mg of naloxone HCl, oxycodone or a pharmaceutically acceptable salt thereof in an amount equivalent to 5 mg of oxycodone HCl and naloxone or a pharmaceutically acceptable salt thereof in an amount equivalent to 2.5 mg of naloxone HCl, oxycodone or a pharmaceutically acceptable salt thereof in an amount equivalent to 7.5 mg of oxycodone HCl and naloxone or a pharmaceutically acceptable salt thereof in an amount equivalent to 3.75 mg of naloxone HCl, oxycodone or a pharmaceutically acceptable salt thereof in an amount equivalent to 10 mg of oxycodone HCl and naloxone or a pharmaceutically acceptable salt thereof in an amount equivalent to 5 mg of naloxone HCl, oxycodone or a pharmaceutically acceptable salt thereof in an amount equivalent to 12.5 mg of oxycodone HCl and naloxone or a pharmaceutically acceptable salt thereof in an amount equivalent to 6.25 mg of naloxone HCl, or oxycodone or a pharmaceutically acceptable salt thereof in an amount equivalent to 15 mg of oxycodone HCl and naloxone or a pharmaceutically acceptable salt thereof in an amount equivalent to 7.5 mg of naloxone HCl.
79. Use according to any of embodiments 76, 77 or 78, wherein the pharmaceutical composition does not comprise povidone as a binder.
80. Use according to any of embodiments 76, 77 or 78, wherein the pharmaceutical composition comprises hydroxypropyl cellulose as a binder.
81. Use according to any of embodiments 76, 77 or 78, wherein the pharmaceutical composition does not comprise hydroxypropyl cellulose as a binder.
82. Use according to any of embodiments 76 to 81, wherein the pharmaceutical composition releases the pharmaceutically active agents with the following in vitro release rate when measured using the Ph. Eur. paddle method in 500 ml of Simulated Gastric Fluid at 50 rpm at 37 degrees C°:

| | |
|---|---|
| at 15 min: | 10 to 30 % by weight of the pharmaceutically active agents, |
| at 2 h: | 40 to 60% by weight of the pharmaceutically active agents, |
| at 10 h: | more than 80% by weight of the pharmaceutically active agents. |

83. Use according to any of embodiments 67 to 75, wherein the pharmaceutical composition comprises oxycodone or a pharmaceutically acceptable salt thereof in an amount equivalent to 10 to 160 mg, preferably 16 to 160 mg of oxycodone HCl and naloxone or a pharmaceutically acceptable salt thereof in an amount equivalent to 5 to 80 mg, preferably 8 to 80 mg of naloxone HCl.
84. Use according to embodiment 83, wherein the pharmaceutical composition comprises oxycodone or a pharmaceutically acceptable salt thereof and naloxone or a pharmaceutically acceptable salt thereof in 2:1 ratio by weight.
85. Use according to embodiment 83 or 84, wherein the prolonged release pharmaceutical composition comprises oxycodone or a pharmaceutically acceptable salt thereof in an amount equivalent to 10 mg of oxycodone HCl and naloxone or a pharmaceutically acceptable salt thereof in an amount equivalent to 5 mg of naloxone HCl, oxycodone or a pharmaceutically acceptable salt thereof in an amount equivalent to 20 mg of oxycodone HCl and naloxone or a pharmaceutically acceptable salt thereof in an amount equivalent to 10 mg of naloxone HCl, oxycodone or a pharmaceutically acceptable salt thereof in an amount equivalent to 30 mg of oxycodone HCl and naloxone or a pharmaceutically acceptable salt thereof in an amount equivalent to 15 mg of naloxone HCl, oxycodone or a pharmaceutically acceptable salt thereof in an amount equivalent to 40 mg of oxycodone HCl and naloxone or a pharmaceutically acceptable salt thereof in an amount equivalent to 20 mg of naloxone HCl, oxycodone or a pharmaceutically acceptable salt thereof in an amount equivalent to 60 mg of oxycodone HCl and naloxone or a pharmaceutically acceptable salt thereof in an amount equivalent to 30 mg of naloxone HCl, oxycodone or a pharmaceutically acceptable salt thereof in an amount equivalent to 80 mg of oxycodone HCl and naloxone or a pharmaceutically acceptable salt thereof in an amount equivalent to 40 mg of naloxone HCl and oxycodone or a pharmaceutically acceptable salt thereof in an amount equivalent to 160 mg of oxycodone HCl and naloxone or a pharmaceutically acceptable salt thereof in an amount equivalent to 80 mg of naloxone HCl.
86. Use according to any of embodiments 83, 84 or 85, wherein the pharmaceutical composition does not comprise povidone as a binder.
87. Use according to any of embodiments 83, 84 or 85, wherein the pharmaceutical composition comprises povidone as a binder.
88. Use according to any of embodiments 83 to 87 wherein the composition releases the pharmaceutically active agents with the following in vitro release rate when measured using the Ph. Eur. paddle method in 900 ml of Simulated Gastric Fluid at 50 rpm at 37 degrees C°:

| | |
|---|---|
| at 15 min: | 10 to 30 % by weight of the pharmaceutically active agents, |
| at 2 h: | 40 to 65% by weight of the pharmaceutically active agents, |
| at 10 h: | more than 80% by weight of the pharmaceutically active agents. |

89. Use of a hydroxypropyl cellulose for manufacturing a prolonged release pharmaceutical composition comprising oxycodone or a pharmaceutically acceptable salt thereof and naloxone or a pharmaceutically acceptable salt thereof wherein the pharmaceutical composition after storage under stressed conditions has less than 4 % of total substances related to oxycodone or a pharmaceutically acceptable salt or derivative thereof and/or related to naloxone or a pharmaceutically acceptable salt or derivative thereof.
90. Use of a hydroxypropyl cellulose for manufacturing a prolonged release pharmaceutical composition comprising oxycodone or a pharmaceutically acceptable salt thereof and naloxone or a pharmaceutically acceptable salt thereof wherein the pharmaceutical composition after storage under stressed conditions has less than 0.5 % of known oxycodone or a pharmaceutically acceptable salt or derivative thereof related substances or known naloxone or a pharmaceutically acceptable salt or derivative thereof related substances.
91. Use of hydroxypropyl cellulose for manufacturing a prolonged release pharmaceutical composition comprising oxycodone or a pharmaceutically acceptable salt thereof and naloxone or a pharmaceutically acceptable salt thereof, wherein the pharmaceutical composition after storage under stressed conditions releases the pharmaceutically active agents with substantially the same release rate as before subjecting the pharmaceutical composition to stressed conditions.
92. Use according to embodiment 89, 90 or 91, wherein the pharmaceutical composition comprises at least part of oxycodone or a pharmaceutically acceptable salt thereof and at least part of naloxone or a pharmaceutically acceptable salt in a prolonged release matrix.
93. Use according to embodiment 92, wherein the prolonged release matrix comprises a prolonged release matrix material being selected from the group comprising hydrophobic or hydrophilic polymers, gums, substituted or unsubstituted hydrocarbons, carbohydrates, fatty acids, fatty alcohols, glyceryl esters of fatty acids, oils and waxes.
94. Use according to embodiment 93, wherein the pharmaceutical composition comprises a prolonged release matrix which comprises at least one cellulose ether, preferably at least one hydrophobic cellulose ether such as ethyl cellulose as prolonged release matrix material.
95. Use according to embodiment 93, wherein the pharmaceutical composition comprises a prolonged release matrix which comprises at least one fatty alcohol as prolonged release material as prolonged release matrix material.
96. Use according to any of embodiments 93, 94, or 95, wherein the pharmaceutical composition comprises a prolonged release matrix which comprises at least one cellulose ether, preferably at least one hydrophobic cellulose ether such as ethyl cellulose and at least one fatty alcohol as prolonged release matrix materials.
97. Use according to any of embodiments 89 to 96, wherein the pharmaceutical composition comprises additionally at least one filler and at least one lubricant.
98. Use according to embodiment 97, wherein said filler is lactose monohydrate.
99. Use according to embodiment 97, wherein magnesium stearate and/or talc are used as lubricants.
100. Use according to any of embodiments 89 to 99, wherein the pharmaceutical composition comprises oxycodone or a pharmaceutically acceptable salt thereof and naloxone or a pharmaceutically acceptable salt thereof in 2:1 ratio by weight.
101. Use according to any of embodiments 89 to 100, wherein the pharmaceutical composition comprises oxycodone or a pharmaceutically acceptable salt thereof in an amount equivalent to 1 to 15 mg, preferably, 1 to 9 mg of oxycodone HCl and naloxone or a pharmaceutically acceptable salt thereof in an amount equivalent to 0.5 to 7.5 mg, preferably 0.5 to 4.5 mg of naloxone HCl.
102. Use according to embodiment 100 or 101, wherein the pharmaceutical composition comprises oxycodone or a pharmaceutically acceptable salt thereof in an amount equivalent to 1 mg of oxycodone HCl and naloxone or a pharmaceutically acceptable salt thereof in an amount equivalent to 0.5 mg of naloxone HCl, oxycodone or a pharmaceutically acceptable salt thereof in an amount equivalent to 2.5 mg of oxycodone HCl and naloxone or a pharmaceutically acceptable salt thereof in an amount equivalent to 1.25 mg of naloxone HCl, oxycodone or a pharmaceutically acceptable salt thereof in an amount equivalent to 5 mg of oxycodone HCl and naloxone or a pharmaceutically acceptable salt thereof in an amount equivalent to 2.5 mg of naloxone HCl, oxycodone or a pharmaceutically acceptable salt thereof in an amount equivalent to 7.5 mg of oxycodone HCl and naloxone in an amount equivalent to 3.75 mg of naloxone HCl, oxycodone or a pharmaceutically acceptable salt thereof in an amount equivalent to 10 mg of oxycodone HCl and naloxone in an amount equivalent to 5 mg of naloxone HCl, oxycodone or a pharmaceutically acceptable salt thereof in an amount equivalent to 12.5 mg of oxycodone HCl and naloxone in an amount equivalent to 6.25 mg of naloxone HCl, or oxycodone or a pharmaceutically acceptable salt thereof in an amount equivalent to 15 mg of oxycodone HCl and naloxone in an amount equivalent to 7.5 mg of naloxone HCl.
103. Use according to any of embodiments 100, 101 or 102, wherein the pharmaceutical composition does not comprise povidone as a binder.
104. Use according to any of embodiments 89 to 103, wherein the pharmaceutical composition releases the pharmaceutically active agents with the following in vitro release rate when measured using the Ph. Eur. paddle method in 500 ml of Simulated Gastric Fluid at 50 rpm at 37 degrees C°:

| | |
|---|---|
| at 15 min: | 10 to 30 % by weight of the pharmaceutically active agents, |
| at 2 h: | 40 to 60% by weight of the pharmaceutically active agents, |
| at 10 h: | more than 80% by weight of the pharmaceutically active agents. |

105. Use of povidone for manufacturing a prolonged release pharmaceutical composition comprising oxycodone or a pharmaceutically acceptable salt thereof and naloxone or a pharmaceutically acceptable salt thereof wherein the pharmaceutical composition after storage under stressed conditions has less than 4 % of total substances related to oxycodone or a pharmaceutically acceptable salt or derivative thereof and/or related to naloxone or a pharmaceutically acceptable salt or derivative thereof.
106. Use of povidone for manufacturing a prolonged release pharmaceutical composition comprising oxycodone or a pharmaceutically acceptable salt thereof and naloxone or a pharmaceutically acceptable salt thereof wherein the pharmaceutical composition after storage under stressed conditions has less than 0.5 % of known oxycodone or a pharmaceutically acceptable salt or derivative thereof related substances or known naloxone or a pharmaceutically acceptable salt or derivative thereof related substances.
107. Use of povidone for manufacturing a prolonged release pharmaceutical composition comprising oxycodone or a pharmaceutically acceptable salt thereof and naloxone or a pharmaceutically acceptable salt thereof, wherein the pharmaceutical composition after storage under stressed conditions releases the pharmaceutically active agents with substantially the same release rate as before subjecting the pharmaceutical composition to stressed conditions.
108. Use according to embodiment 105, 106, or 107, wherein the pharmaceutical composition comprises at least part of oxycodone or a pharmaceutically acceptable salt thereof and at least part of naloxone or a pharmaceutically acceptable salt in a prolonged release matrix.
109. Use according to embodiment 108, wherein the prolonged release matrix comprises a prolonged release matrix material being selected from the group comprising hydrophobic or hydrophilic polymers, gums, substituted or unsubstituted hydrocarbons, carbohydrates, fatty acids, fatty alcohols, glyceryl esters of fatty acids, oils and waxes.
110. Use according to embodiment 109, wherein the pharmaceutical composition comprises a prolonged release matrix which comprises at least one cellulose ether, preferably at least one hydrophobic cellulose ether such as ethyl cellulose as prolonged release matrix material.
111. Use according to embodiment 109, wherein the pharmaceutical composition comprises a prolonged release matrix which comprises at least one fatty alcohol as prolonged release material as prolonged release matrix material.
112. Use according to any of embodiments 109 to 111, wherein the pharmaceutical composition comprises a prolonged release matrix which comprises at least one cellulose ether, preferably at least one hydrophobic cellulose ether such as ethyl cellulose and at least one fatty alcohol as prolonged release matrix materials.
113. Use according to any of embodiments 105 to 112, wherein the pharmaceutical composition comprises additionally at least one filler and at least one lubricant.
114. Use according to embodiment 113, wherein said filler is lactose monohydrate.
115. Use according to embodiment 113, wherein magnesium stearate and/or talc are used as lubricants.
116. Use according to any of embodiments 105 to 115, wherein the pharmaceutical composition comprises oxycodone or a pharmaceutically acceptable salt thereof and naloxone or a pharmaceutically acceptable salt thereof in 2:1 ratio by weight.
117. Use according to any of embodiments 105 to 116, wherein the pharmaceutical composition comprises oxycodone or a pharmaceutically acceptable salt thereof in an amount equivalent to 10 to 160 mg, preferably 16 to 160 mg of oxycodone HCl and naloxone or a pharmaceutically acceptable salt thereof in an amount equivalent to 5 to 80 mg, preferably 8 to 80 mg of naloxone HCl.
118. Use according to embodiment 116 or 117, wherein the prolonged release pharmaceutical composition comprises oxycodone or a pharmaceutically acceptable salt thereof in an amount equivalent to 10 mg of oxycodone HCl and naloxone or a pharmaceutically acceptable salt thereof in an amount equivalent to 5 mg of naloxone HCl, oxycodone or a pharmaceutically acceptable salt thereof in an amount equivalent to 20 mg of oxycodone HCl and naloxone or a pharmaceutically acceptable salt thereof in an amount equivalent to 10 mg of naloxone HCl, oxycodone or a pharmaceutically acceptable salt thereof in an amount equivalent to 30 mg of oxycodone HCl and naloxone or a pharmaceutically acceptable salt thereof in an amount equivalent to 15 mg of naloxone HCl, oxycodone or a pharmaceutically acceptable salt thereof in an amount equivalent to 40 mg of oxycodone HCl and naloxone or a pharmaceutically acceptable salt thereof in an amount equivalent to 20 mg of naloxone HCl, oxycodone or a pharmaceutically acceptable salt thereof in an amount equivalent to 60 mg of oxycodone HCl and naloxone or a pharmaceutically acceptable salt thereof in an amount equivalent to 30 mg of naloxone HCl, oxycodone or a pharmaceutically acceptable salt thereof in an amount equivalent to 80 mg of oxycodone HCl and naloxone or a pharmaceutically acceptable salt thereof in an amount equivalent to 40 mg of naloxone HCl, and oxycodone or a pharmaceutically acceptable salt thereof in an amount equivalent to 160 mg of oxycodone HCl and naloxone or a pharmaceutically acceptable salt thereof in an amount equivalent to 80 mg of naloxone HCl.
119. Use according to any of embodiments 105 to 118, wherein the pharmaceutical composition releases the pharmaceutically active agents with the following in vitro release rate when measured using the Ph. Eur. paddle method in 900 ml of Simulated Gastric Fluid at 50 rpm at 37 degrees C°:

| | |
|---|---|
| at 15 min: | 10 to 30 % by weight of the pharmaceutically active agents, |
| at 2 h: | 40 to 65% by weight of the pharmaceutically active agents, |
| at 10 h: | more than 80% by weight of the pharmaceutically active agents. |

120. Use of HPC for stabilizing a prolonged release pharmaceutical composition comprising oxycodone or a pharmaceutically acceptable salt thereof and naloxone or a pharmaceutically acceptable salt thereof.
121. Use according to embodiment 120, wherein the pharmaceutical composition comprises at least part of oxycodone or a pharmaceutically acceptable salt thereof and of naloxone or a pharmaceutically acceptable salt thereof in a prolonged release matrix.
122. Use according to any of embodiments 120 or 121, wherein the pharmaceutical composition comprises oxycodone or a pharmaceutically acceptable salt thereof in an amount equivalent to 1 to 15 mg, preferably 1 to 9 mg of oxycodone HCl and naloxone or a pharmaceutically acceptable salt thereof in an amount equivalent to 0.5 to 7.5 mg, preferably 0.5 to 4.5 mg of naloxone HCl.
123. Use according to any of embodiments 120, 121 or 122, wherein the pharmaceutical composition comprises oxycodone or a pharmaceutically acceptable salt thereof and naloxone or a pharmaceutically acceptable salt thereof in 2:1 ratio by weight.
124. An oral prolonged release pharmaceutical composition comprising at least:
a) at least one prolonged release material;
b) at least oxycodone or a pharmaceutically acceptable salt or derivative thereof and naloxone or a pharmaceutically acceptable salt or derivative thereof; and
c) HPC as a binder.
125. Pharmaceutical composition according to embodiment 124, wherein the prolonged release pharmaceutical composition comprises oxycodone or a pharmaceutically acceptable salt thereof in an amount equivalent to 1 to 80 mg, of oxycodone HCl and naloxone or a pharmaceutically acceptable salt thereof in an amount equivalent to 0.5 to 40 mg of naloxone HCl.
126. Pharmaceutical composition according to embodiment 124 or 125, wherein the prolonged release pharmaceutical composition comprises oxycodone or a pharmaceutically acceptable salt thereof and naloxone or a pharmaceutically acceptable salt thereof in a ratio of 2:1 by weight.
127. Pharmaceutical composition according to embodiment 124, 125, or 126, wherein the prolonged release pharmaceutical composition comprises oxycodone or a pharmaceutically acceptable salt thereof in an amount equivalent to 1 mg of oxycodone HCl and naloxone or a pharmaceutically acceptable salt thereof in an amount equivalent to 0.5 mg of naloxone HCl, oxycodone or a pharmaceutically acceptable salt thereof in an amount equivalent to 2.5 mg of oxycodone HCl and naloxone or a pharmaceutically acceptable salt thereof in an amount equivalent to 1.25 mg of naloxone HCl, oxycodone or a pharmaceutically acceptable salt thereof in an amount equivalent to 5 mg of oxycodone HCl and naloxone or a pharmaceutically acceptable salt thereof in an amount equivalent to 2.5 mg of naloxone HCl, oxycodone or a pharmaceutically acceptable salt thereof in an amount equivalent to 7.5 mg of oxycodone HCl and naloxone in an amount equivalent to 3.75 mg of naloxone HCl, oxycodone or a pharmaceutically acceptable salt thereof in an amount equivalent to 10 mg of oxycodone HCl and naloxone in an amount equivalent to 5 mg of naloxone HCl, oxycodone or a pharmaceutically acceptable salt thereof in an amount equivalent to 12.5 mg of oxycodone HCl and naloxone in an amount equivalent to 6.25 mg of naloxone HCl, oxycodone or a pharmaceutically acceptable salt thereof in an amount equivalent to 15 mg of oxycodone HCl and naloxone in an amount equivalent to 7.5 mg of naloxone HCl, oxycodone or a pharmaceutically acceptable salt thereof in an amount equivalent to 20 mg of oxycodone HCl and naloxone in an amount equivalent to 10 mg of naloxone HCl, oxycodone or a pharmaceutically acceptable salt thereof in an amount equivalent to 30 mg of oxycodone HCl and naloxone in an amount equivalent to 15 mg of naloxone HCl, oxycodone or a pharmaceutically acceptable salt thereof in an amount equivalent to 40 mg of oxycodone HCl and naloxone in an amount equivalent to 20 mg of naloxone HCl, oxycodone or a pharmaceutically acceptable salt thereof in an amount equivalent to 60 mg of oxycodone HCl and naloxone in an amount equivalent to 30 mg of naloxone HCl, or oxycodone or a pharmaceutically acceptable salt thereof in an amount equivalent to 80 mg of oxycodone HCl and naloxone in an amount equivalent to 40 mg of naloxone HCl.
128. Pharmaceutical composition according to any of embodiments 124 to 127, wherein the at least one prolonged release material and oxycodone or a pharmaceutically acceptable salt thereof and naloxone or a pharmaceutically acceptable salt thereof are combined to form a prolonged release matrix.
129. Pharmaceutical composition according to embodiment 128, wherein the prolonged release material is selected from the group comprising hydrophobic or hydrophilic polymers, gums, substituted or unsubstituted hydrocarbons, carbohydrates, fatty acids, fatty alcohols, glyceryl esters of fatty acids, natural and synthetic oils and waxes.
130. Pharmaceutical composition according to embodiment 129, wherein the prolonged release material is a cellulose ether, a (meth)acrylic based (co)polymer and/or a fatty alcohol.
131. Pharmaceutical composition according to any of embodiments 128, 129 or 130, comprising at least:
a) at least one cellulose ether, preferably at least one hydrophobic cellulose ether such as ethyl cellulose as prolonged release material;
b) at least oxycodone or a pharmaceutically acceptable salt or derivative thereof and naloxone or a pharmaceutically acceptable salt or derivative thereof, wherein
c) oxycodone or a pharmaceutically acceptable salt or derivative thereof and naloxone or a pharmaceutically acceptable salt or derivative thereof are combined with said prolonged release material to form a prolonged release matrix.
132. Pharmaceutical composition according to any of embodiments 128, 129 or 130, comprising at least:
a) at least one fatty alcohol as prolonged release material;
b) at least oxycodone or a pharmaceutically acceptable salt or derivative thereof and naloxone or a pharmaceutically acceptable salt or derivative thereof, wherein
c) oxycodone or a pharmaceutically acceptable salt or derivative thereof and naloxone or a pharmaceutically acceptable salt or derivative thereof are combined with said prolonged release material to form a prolonged release matrix.
133. Pharmaceutical composition according to any of embodiments 128, 129
or 130, comprising at least:
a) at least one cellulose ether, preferably at least one hydrophobic cellulose ether such as ethyl cellulose and at least one fatty alcohol as prolonged release materials;
b) at least oxycodone or a pharmaceutically acceptable salt or derivative thereof and naloxone or a pharmaceutically acceptable salt or derivative thereof, wherein
c) oxycodone or a pharmaceutically acceptable salt or derivative thereof and naloxone or a pharmaceutically acceptable salt or derivative thereof are combined with said prolonged release materials to form a prolonged release matrix.
134. Pharmaceutical composition according to any of embodiments 128, 129, 130, 131, 132, or 133, wherein the pharmaceutical composition comprises additionally at least one filler and at least one lubricant.
135. Pharmaceutical composition according to embodiment 134, wherein said filler is lactose monohydrate.
136. Pharmaceutical composition according to embodiment 134, wherein magnesium stearate and/or talc are used as lubricants.
137. Pharmaceutical composition according to any of embodiments 124 to 136, wherein the composition releases the pharmaceutically active agents with the following in vitro release rate when measured using the Ph. Eur. paddle method in 500 ml of Simulated Gastric Fluid at 50 rpm at 37 degrees C°:

| | |
|---|---|
| at 15 min: | 10 to 30 % by weight of the pharmaceutically active agents, |
| at 2 h: | 40 to 65% by weight of the pharmaceutically active agents, |
| at 10 h: | more than 80% by weight of the pharmaceutically active agents. |

138. Use of HPC as a binder to produce a prolonged release formulation in accordance with any of embodiments 124 to 138.

## Claims

1. Use of hydroxypropyl cellulose for stabilizing a prolonged release pharmaceutical composition comprising oxycodone or a pharmaceutically acceptable salt thereof and naloxone or a pharmaceutically acceptable salt thereof.

2. The use according to claim 1, wherein the pharmaceutical composition comprises at least part of oxycodone or a pharmaceutically acceptable salt thereof and of naloxone or a pharmaceutically acceptable salt thereof in a prolonged release matrix.

3. The use according to claim 2, wherein the prolonged release matrix does not comprise ethylcellulose and/or stearyl alcohol as prolonged release matrix materials

4. The use according to any one of claims 1 to 3, wherein the pharmaceutical composition comprises oxycodone or a pharmaceutically acceptable salt thereof in an amount equivalent to 1 to 15 mg, preferably 1 to 9 mg of oxycodone HCl and naloxone or a pharmaceutically acceptable salt thereof in an amount equivalent to 0.5 to 7.5 mg, preferably 0.5 to 4.5 mg of naloxone HCl.

5. The use according to any one of claims 1 to 4, wherein the pharmaceutical composition comprises oxycodone or a pharmaceutically acceptable salt thereof and naloxone or a pharmaceutically acceptable salt thereof in 2:1 ratio by weight.

6. The use according to any one of claims 1 to 5, wherein the pharmaceutical composition comprises oxycodone HCl and naloxone HCl.

7. The use according to any one of claims 1 to 3, wherein the pharmaceutical composition comprises oxycodone or a pharmaceutically acceptable salt thereof in an amount equivalent to 10 to 160 mg, preferably 16 to 160 mg of oxycodone HCl and naloxone or a pharmaceutically acceptable salt thereof in an amount equivalent to 5 to 80 mg, preferably 8 to 80 mg of naloxone HCl.

8. The use according to claim 7, wherein the pharmaceutical composition comprises oxycodone or a pharmaceutically acceptable salt thereof and naloxone or a pharmaceutically acceptable salt thereof in 2:1 ratio by weight.

9. The use according to claim 7 or 8, wherein the pharmaceutical composition comprises oxycodone HCl and naloxone HCl.

10. The use according to any one of claims 1 to 9, wherein the pharmaceutical composition does not comprise povidone as a binder.

11. The use according to claim 1, wherein hydroxypropyl cellulose is used as binder.

12. The use according to claim 11, wherein hydroxypropyl cellulose is used as binder in an amount of 1% to 10% by weight based on the weight of the pharmaceutical composition.

13. The use according to claim 11, wherein hydroxypropyl cellulose is used as binder in an amount of 3.0% to 4.0% by weight based on the weight of the pharmaceutical composition.
